# DEMANDE DE BREVET EUROPEEN

(11) **EP 1 006 123 A2**
(43) Date de publication de la demande: **07.06.2000**
(21) Numéro de dépôt: 99400735.9
(22) Date de dépôt: 25.03.1999
(51) Int. Cl.: C07K 14/15, C12N 15/867, C12N 15/62, A61K 48/00

(54) **Protéines d'enveloppe, methodes et utilisations**

(30) Priorité: 03.12.1998 FR 9815302
(71) Demandeur: UNIVERSITE PIERRE ET MARIE CURIE PARIS VI, 75252 Paris Cédex 05 (FR)
(72) Inventeur: Klatzmann, David, 75013, Paris (FR); Salzmann, Jean-Loup, 75005, Paris (FR); Noguiez, Patricia, 91360 Guibeville (FR); Cosset, François Loic, 69007, Lyon (FR)
(74) Mandataire: Becker, Philippe

(57) **Abrégé**

La présente invention concerne de protéines et compositions utilisables pour la production de virus, notamment rétrovirus recombinants. Elle concerne également des acides nucléiques, vecteurs et cellules modifiées, ainsi que leurs utilisations. L'invention est plus particulièrement relative à de protéines dérivées du virus GALV et leurs utilisations.

La présente invention concerne en particulier une protéine d'enveloppe chimérique caractérisée en ce qu'elle comprend au moins une partie du domaine extracellulaire de l'enveloppe du virus GALV ou d'un variant fonctionnel de celui-ci.

LISTE DE SEQUENCES

<110> UNIVERSITE PIERRE ET MARIE CURIE (PARIS VI)

<120> PROTEINES D'ENVELOPPE, METHODES ET UTILISATIONS

<130> B4124A - PB/KM

<140>
<141>

<160> 14

<170> PatentIn Ver. 2.1

<210> 1
<211> 2129
<212> ADN
<213> Séquence artificielle

<220>
<223> Description de la séquence artificielle: ENVELOPPE
      GALV

<220>
<221> CDS
<222> (1)..(2058)

<400> 1
atg gta ttg ctg cct ggg tcc atg ctt ctc acc tca aac ctg cac cac    48
Met Val Leu Leu Pro Gly Ser Met Leu Leu Thr Ser Asn Leu His His
  1               5                  10                  15

ctt cgg cac cag atg agt cct ggg agc tgg aaa aga ctg atc atc ctc    96
Leu Arg His Gln Met Ser Pro Gly Ser Trp Lys Arg Leu Ile Ile Leu
             20                  25                  30

tta agc tgc gta ttc ggc ggc ggc ggg acg agt ctg caa aat aag aac    144
Leu Ser Cys Val Phe Gly Gly Gly Gly Thr Ser Leu Gln Asn Lys Asn
         35                  40                  45

ccc cac cag ccc atg acc ctc act tgg cag gta ctg tcc caa act gga    192
Pro His Gln Pro Met Thr Leu Thr Trp Gln Val Leu Ser Gln Thr Gly
     50                  55                  60

gac gtt gtc tgg gat aca aag gca gtc cag ccc cct tgg act tgg tgg    240
Asp Val Val Trp Asp Thr Lys Ala Val Gln Pro Pro Trp Thr Trp Trp
 65                  70                  75                  80

ccc aca ctt aaa cct gat gta tgt gcc ttg gcg gct agt ctt gag tcc    288
Pro Thr Leu Lys Pro Asp Val Cys Ala Leu Ala Ala Ser Leu Glu Ser
                 85                  90                  95

tgg gat atc ccg gga acc gat gtc tcg tcc tct aaa cga gtc aga cct    336
Trp Asp Ile Pro Gly Thr Asp Val Ser Ser Ser Lys Arg Val Arg Pro
            100                 105                 110

ccg gac tca gac tat act gcc gct tat aag caa atc acc tgg gga gcc    384

Pro Asp Ser Asp Tyr Thr Ala Ala Tyr Lys Gln Ile Thr Trp Gly Ala
        115                 120                 125

ata ggg tgc agc tac cct cgg gct agg act aga atg gca agc tct acc     432
Ile Gly Cys Ser Tyr Pro Arg Ala Arg Thr Arg Met Ala Ser Ser Thr
    130                 135                 140

ttc tac gta tgt ccc cgg gat ggc cgg acc ctt tca gaa gct aga agg     480
Phe Tyr Val Cys Pro Arg Asp Gly Arg Thr Leu Ser Glu Ala Arg Arg
145                 150                 155                 160

tgc ggg ggg cta gaa tcc cta tac tgt aaa gaa tgg gat tgt gag acc     528
Cys Gly Gly Leu Glu Ser Leu Tyr Cys Lys Glu Trp Asp Cys Glu Thr
                165                 170                 175

acg ggg acc ggt tat tgg cta tct aaa tcc tca aaa gac ctc ata act     576
Thr Gly Thr Gly Tyr Trp Leu Ser Lys Ser Ser Lys Asp Leu Ile Thr
            180                 185                 190

gta aaa tgg gac caa aat agc gaa tgg act caa aaa ttt caa cag tgt     624
Val Lys Trp Asp Gln Asn Ser Glu Trp Thr Gln Lys Phe Gln Gln Cys
        195                 200                 205

cac cag acc ggc tgg tgt aac ccc ctt aaa ata gat ttc aca gac aaa     672
His Gln Thr Gly Trp Cys Asn Pro Leu Lys Ile Asp Phe Thr Asp Lys
    210                 215                 220

gga aaa tta tcc aag gac tgg ata acg gga aaa acc tgg gga tta aga     720
Gly Lys Leu Ser Lys Asp Trp Ile Thr Gly Lys Thr Trp Gly Leu Arg
225                 230                 235                 240

ttc tat gtg tct gga cat cca ggc gta cag ttc acc att cgc tta aaa     768
Phe Tyr Val Ser Gly His Pro Gly Val Gln Phe Thr Ile Arg Leu Lys
                245                 250                 255

atc acc aac atg cca gct gtg gca gta ggt cct gac ctc gtc ctt gtg     816
Ile Thr Asn Met Pro Ala Val Ala Val Gly Pro Asp Leu Val Leu Val
            260                 265                 270

gaa caa gga cct cct aga acg tcc ctc gct ctc cca cct cct ctt ccc     864
Glu Gln Gly Pro Pro Arg Thr Ser Leu Ala Leu Pro Pro Pro Leu Pro
        275                 280                 285

cca agg gaa gcg cca ccg cca tct ctc ccc gac tct aac tcc aca gcc     912
Pro Arg Glu Ala Pro Pro Pro Ser Leu Pro Asp Ser Asn Ser Thr Ala
    290                 295                 300

ctg gcg act agt gca caa act ccc acg gtg aga aaa aca att gtt acc     960
Leu Ala Thr Ser Ala Gln Thr Pro Thr Val Arg Lys Thr Ile Val Thr
305                 310                 315                 320

cta aac act ccg cct ccc acc aca ggc gac aga ctt ttt gat ctt gtg    1008
Leu Asn Thr Pro Pro Pro Thr Thr Gly Asp Arg Leu Phe Asp Leu Val
                325                 330                 335

cag ggg gcc ttc cta acc tta aat gct acc aac cca ggg gcc act gag    1056
Gln Gly Ala Phe Leu Thr Leu Asn Ala Thr Asn Pro Gly Ala Thr Glu
            340                 345                 350

tct tgc tgg ctt tgt ttg gcc atg ggc ccc cct tat tat gaa gca ata    1104
Ser Cys Trp Leu Cys Leu Ala Met Gly Pro Pro Tyr Tyr Glu Ala Ile
        355                 360                 365

gcc tca tca gga gag gtc gcc tac tcc acc gac ctt gac cgg tgc cgc    1152
Ala Ser Ser Gly Glu Val Ala Tyr Ser Thr Asp Leu Asp Arg Cys Arg
    370                 375                 380

tgg ggg acc caa gga aag ctc acc ctc act gag gtc tca gga cac ggg    1200
Trp Gly Thr Gln Gly Lys Leu Thr Leu Thr Glu Val Ser Gly His Gly
385                 390                 395                 400

ttg tgc ata gga aag gtg ccc ttt acc cat cag cat ctc tgc aat cag    1248
Leu Cys Ile Gly Lys Val Pro Phe Thr His Gln His Leu Cys Asn Gln
                405                 410                 415

acc cta tcc atc aat tcc tcc gga gac cat cag tat ctg ctc ccc tcc    1296
Thr Leu Ser Ile Asn Ser Ser Gly Asp His Gln Tyr Leu Leu Pro Ser
            420                 425                 430

aac cat agc tgg tgg gct tgc agc act ggc ctc acc cct tgc ctc tcc    1344
Asn His Ser Trp Trp Ala Cys Ser Thr Gly Leu Thr Pro Cys Leu Ser
        435                 440                 445

acc tca gtt ttt aat cag act aga gat ttc tgt atc cag gtc cag ctg    1392
Thr Ser Val Phe Asn Gln Thr Arg Asp Phe Cys Ile Gln Val Gln Leu
    450                 455                 460

att cct cgc atc tat tac tat cct gaa gaa gtt ttg tta cag gcc tat    1440
Ile Pro Arg Ile Tyr Tyr Tyr Pro Glu Glu Val Leu Leu Gln Ala Tyr
465                 470                 475                 480

gac aat tct cac ccc agg act aaa aga gag gct gtc tca ctt acc cta    1488
Asp Asn Ser His Pro Arg Thr Lys Arg Glu Ala Val Ser Leu Thr Leu
                485                 490                 495

gct gtt tta ctg ggg ttg gga atc acg gcg gga ata ggt act ggt tca    1536
Ala Val Leu Leu Gly Leu Gly Ile Thr Ala Gly Ile Gly Thr Gly Ser
            500                 505                 510

act gcc tta att aaa gga cct ata gac ctc cag caa ggc ctg aca agc    1584
Thr Ala Leu Ile Lys Gly Pro Ile Asp Leu Gln Gln Gly Leu Thr Ser
        515                 520                 525

ctc cag atc gcc ata gat gct gac ctc cgg gcc ctc caa gac tca gtc    1632
Leu Gln Ile Ala Ile Asp Ala Asp Leu Arg Ala Leu Gln Asp Ser Val
    530                 535                 540

agc aag tta gag gac tca ctg act tcc ctg tcc gag gta gtg ctc caa    1680
Ser Lys Leu Glu Asp Ser Leu Thr Ser Leu Ser Glu Val Val Leu Gln
545                 550                 555                 560

aat agg aga ggc ctt gac ttg ctg ttt cta aaa gaa ggt ggc ctc tgt    1728
Asn Arg Arg Gly Leu Asp Leu Leu Phe Leu Lys Glu Gly Gly Leu Cys
                565                 570                 575

gcg gcc cta aag gaa gag tgc tgt ttt tac ata gac cac tca ggt gca    1776

Ala Ala Leu Lys Glu Glu Cys Cys Phe Tyr Ile Asp His Ser Gly Ala
            580                 585                 590

gta cgg gac tcc atg aaa aaa ctc aaa gaa aaa ctg gat aaa aga cag    1824
Val Arg Asp Ser Met Lys Lys Leu Lys Glu Lys Leu Asp Lys Arg Gln
        595                 600                 605

tta gag cgc cag aaa agc caa aac tgg tat gaa gga tgg ttc aat aac    1872
Leu Glu Arg Gln Lys Ser Gln Asn Trp Tyr Glu Gly Trp Phe Asn Asn
    610                 615                 620

tcc cct tgg ttc act acc ctg cta tca acc atc gct ggg ccc cta tta    1920
Ser Pro Trp Phe Thr Thr Leu Leu Ser Thr Ile Ala Gly Pro Leu Leu
625                 630                 635                 640

ctc ctc ctt ctg ttg ctc atc ctc ggg cca tgc atc atc aat aag tta    1968
Leu Leu Leu Leu Leu Leu Ile Leu Gly Pro Cys Ile Ile Asn Lys Leu
                645                 650                 655

gtt caa ttc atc aat gat agg ata agt gca gtt aaa att ctg gtc ctt    2016
Val Gln Phe Ile Asn Asp Arg Ile Ser Ala Val Lys Ile Leu Val Leu
            660                 665                 670

aga caa aaa tat cag gcc cta gag aac gaa ggt aac ctt taa            2058
Arg Gln Lys Tyr Gln Ala Leu Glu Asn Glu Gly Asn Leu
        675                 680                 685

ttttgctcta agattagagc tattcacaag agaaatgggg gaatgaaaga agtgtttttt 2118

tttagccaac t                                                      2129

<210> 2
<211> 685
<212>
<213> Séquence artificielle
<223> Description de la séquence artificielle: ENVELOPPE
      GALV

<400> 2
Met Val Leu Leu Pro Gly Ser Met Leu Leu Thr Ser Asn Leu His His
  1               5                  10                  15

Leu Arg His Gln Met Ser Pro Gly Ser Trp Lys Arg Leu Ile Ile Leu
             20                  25                  30

Leu Ser Cys Val Phe Gly Gly Gly Gly Thr Ser Leu Gln Asn Lys Asn
         35                  40                  45

Pro His Gln Pro Met Thr Leu Thr Trp Gln Val Leu Ser Gln Thr Gly
     50                  55                  60

Asp Val Val Trp Asp Thr Lys Ala Val Gln Pro Pro Trp Thr Trp Trp
 65                  70                  75                  80

Pro Thr Leu Lys Pro Asp Val Cys Ala Leu Ala Ala Ser Leu Glu Ser
                 85                  90                  95

Trp Asp Ile Pro Gly Thr Asp Val Ser Ser Ser Lys Arg Val Arg Pro
            100                 105                 110

Pro Asp Ser Asp Tyr Thr Ala Ala Tyr Lys Gln Ile Thr Trp Gly Ala
        115                 120                 125

Ile Gly Cys Ser Tyr Pro Arg Ala Arg Thr Arg Met Ala Ser Ser Thr
    130                 135                 140

Phe Tyr Val Cys Pro Arg Asp Gly Arg Thr Leu Ser Glu Ala Arg Arg
145                 150                 155                 160

Cys Gly Gly Leu Glu Ser Leu Tyr Cys Lys Glu Trp Asp Cys Glu Thr
                165                 170                 175

Thr Gly Thr Gly Tyr Trp Leu Ser Lys Ser Ser Lys Asp Leu Ile Thr
            180                 185                 190

Val Lys Trp Asp Gln Asn Ser Glu Trp Thr Gln Lys Phe Gln Gln Cys
        195                 200                 205

His Gln Thr Gly Trp Cys Asn Pro Leu Lys Ile Asp Phe Thr Asp Lys
    210                 215                 220

Gly Lys Leu Ser Lys Asp Trp Ile Thr Gly Lys Thr Trp Gly Leu Arg
225                 230                 235                 240

Phe Tyr Val Ser Gly His Pro Gly Val Gln Phe Thr Ile Arg Leu Lys
                245                 250                 255

Ile Thr Asn Met Pro Ala Val Ala Val Gly Pro Asp Leu Val Leu Val
            260                 265                 270

Glu Gln Gly Pro Pro Arg Thr Ser Leu Ala Leu Pro Pro Pro Leu Pro
        275                 280                 285

Pro Arg Glu Ala Pro Pro Pro Ser Leu Pro Asp Ser Asn Ser Thr Ala
    290                 295                 300

Leu Ala Thr Ser Ala Gln Thr Pro Thr Val Arg Lys Thr Ile Val Thr
305                 310                 315                 320

Leu Asn Thr Pro Pro Pro Thr Thr Gly Asp Arg Leu Phe Asp Leu Val
                325                 330                 335

Gln Gly Ala Phe Leu Thr Leu Asn Ala Thr Asn Pro Gly Ala Thr Glu
            340                 345                 350

Ser Cys Trp Leu Cys Leu Ala Met Gly Pro Pro Tyr Tyr Glu Ala Ile
        355                 360                 365

Ala Ser Ser Gly Glu Val Ala Tyr Ser Thr Asp Leu Asp Arg Cys Arg
    370                 375                 380

Trp Gly Thr Gln Gly Lys Leu Thr Leu Thr Glu Val Ser Gly His Gly
385                 390                 395                 400

Leu Cys Ile Gly Lys Val Pro Phe Thr His Gln His Leu Cys Asn Gln

                    405                 410                 415

Thr Leu Ser Ile Asn Ser Ser Gly Asp His Gln Tyr Leu Leu Pro Ser
            420                 425                 430

Asn His Ser Trp Trp Ala Cys Ser Thr Gly Leu Thr Pro Cys Leu Ser
        435                 440                 445

Thr Ser Val Phe Asn Gln Thr Arg Asp Phe Cys Ile Gln Val Gln Leu
    450                 455                 460

Ile Pro Arg Ile Tyr Tyr Tyr Pro Glu Glu Val Leu Leu Gln Ala Tyr
465                 470                 475                 480

Asp Asn Ser His Pro Arg Thr Lys Arg Glu Ala Val Ser Leu Thr Leu
                485                 490                 495

Ala Val Leu Leu Gly Leu Gly Ile Thr Ala Gly Ile Gly Thr Gly Ser
            500                 505                 510

Thr Ala Leu Ile Lys Gly Pro Ile Asp Leu Gln Gln Gly Leu Thr Ser
        515                 520                 525

Leu Gln Ile Ala Ile Asp Ala Asp Leu Arg Ala Leu Gln Asp Ser Val
    530                 535                 540

Ser Lys Leu Glu Asp Ser Leu Thr Ser Leu Ser Glu Val Val Leu Gln
545                 550                 555                 560

Asn Arg Arg Gly Leu Asp Leu Leu Phe Leu Lys Glu Gly Gly Leu Cys
                565                 570                 575

Ala Ala Leu Lys Glu Glu Cys Cys Phe Tyr Ile Asp His Ser Gly Ala
            580                 585                 590

Val Arg Asp Ser Met Lys Lys Leu Lys Glu Lys Leu Asp Lys Arg Gln
        595                 600                 605

Leu Glu Arg Gln Lys Ser Gln Asn Trp Tyr Glu Gly Trp Phe Asn Asn
    610                 615                 620

Ser Pro Trp Phe Thr Thr Leu Leu Ser Thr Ile Ala Gly Pro Leu Leu
625                 630                 635                 640

Leu Leu Leu Leu Leu Leu Ile Leu Gly Pro Cys Ile Ile Asn Lys Leu
                645                 650                 655

Val Gln Phe Ile Asn Asp Arg Ile Ser Ala Val Lys Ile Leu Val Leu
            660                 665                 670

Arg Gln Lys Tyr Gln Ala Leu Glu Asn Glu Gly Asn Leu
        675                 680                 685


<210> 3
<211> 19
<212> PRT
<213> Séquence artificielle

<220>
<223> Description de la séquence artificielle: Extrémité
      C-terminale enveloppe GALV

<400> 3
Val Lys Ile Leu Val Leu Arg Gln Lys Tyr Gln Ala Leu Glu Asn Glu
  1               5                  10                  15

Gly Asn Leu


<210> 4
<211> 31
<212> PRT
<213> Séquence artificielle

<220>
<223> Description de la séquence artificielle: Domaine
      cytoplasmique enveloppe GALV

<400> 4
Lys Leu Val Gln Phe Ile Asn Asp Arg Ile Ser Ala Val Lys Ile Leu
                  5                  10                  15

Val Leu Arg Gln Lys Tyr Gln Ala Leu Glu Asn Glu Gly Asn Leu
             20                  25                  30


<210> 5
<211> 2129
<212> ADN
<213> Séquence artificielle

<220>
<223> Description de la séquence artificielle: Enveloppe
      GALV

<220>
<221> CDS
<222> (1)..(2004)

<400> 5
atg gta ttg ctg cct ggg tcc atg ctt ctc acc tca aac ctg cac cac    48
Met Val Leu Leu Pro Gly Ser Met Leu Leu Thr Ser Asn Leu His His
  1               5                  10                  15

ctt cgg cac cag atg agt cct ggg agc tgg aaa aga ctg atc atc ctc    96
Leu Arg His Gln Met Ser Pro Gly Ser Trp Lys Arg Leu Ile Ile Leu
             20                  25                  30

tta agc tgc gta ttc ggc ggc ggc ggg acg agt ctg caa aat aag aac    144
Leu Ser Cys Val Phe Gly Gly Gly Gly Thr Ser Leu Gln Asn Lys Asn
         35                  40                  45

ccc cac cag ccc atg acc ctc act tgg cag gta ctg tcc caa act gga    192
Pro His Gln Pro Met Thr Leu Thr Trp Gln Val Leu Ser Gln Thr Gly
     50                  55                  60

gac gtt gtc tgg gat aca aag gca gtc cag ccc cct tgg act tgg tgg 240
Asp Val Val Trp Asp Thr Lys Ala Val Gln Pro Pro Trp Thr Trp Trp
65                  70                  75                  80

ccc aca ctt aaa cct gat gta tgt gcc ttg gcg gct agt ctt gag tcc 288
Pro Thr Leu Lys Pro Asp Val Cys Ala Leu Ala Ala Ser Leu Glu Ser
                85                  90                  95

tgg gat atc ccg gga acc gat gtc tcg tcc tct aaa cga gtc aga cct 336
Trp Asp Ile Pro Gly Thr Asp Val Ser Ser Ser Lys Arg Val Arg Pro
            100                 105                 110

ccg gac tca gac tat act gcc gct tat aag caa atc acc tgg gga gcc 384
Pro Asp Ser Asp Tyr Thr Ala Ala Tyr Lys Gln Ile Thr Trp Gly Ala
        115                 120                 125

ata ggg tgc agc tac cct cgg gct agg act aga atg gca agc tct acc 432
Ile Gly Cys Ser Tyr Pro Arg Ala Arg Thr Arg Met Ala Ser Ser Thr
    130                 135                 140

ttc tac gta tgt ccc cgg gat ggc cgg acc ctt tca gaa gct aga agg 480
Phe Tyr Val Cys Pro Arg Asp Gly Arg Thr Leu Ser Glu Ala Arg Arg
145                 150                 155                 160

tgc ggg ggg cta gaa tcc cta tac tgt aaa gaa tgg gat tgt gag acc 528
Cys Gly Gly Leu Glu Ser Leu Tyr Cys Lys Glu Trp Asp Cys Glu Thr
                165                 170                 175

acg ggg acc ggt tat tgg cta tct aaa tcc tca aaa gac ctc ata act 576
Thr Gly Thr Gly Tyr Trp Leu Ser Lys Ser Ser Lys Asp Leu Ile Thr
            180                 185                 190

gta aaa tgg gac caa aat agc gaa tgg act caa aaa ttt caa cag tgt 624
Val Lys Trp Asp Gln Asn Ser Glu Trp Thr Gln Lys Phe Gln Gln Cys
        195                 200                 205

cac cag acc ggc tgg tgt aac ccc ctt aaa ata gat ttc aca gac aaa 672
His Gln Thr Gly Trp Cys Asn Pro Leu Lys Ile Asp Phe Thr Asp Lys
    210                 215                 220

gga aaa tta tcc aag gac tgg ata acg gga aaa acc tgg gga tta aga 720
Gly Lys Leu Ser Lys Asp Trp Ile Thr Gly Lys Thr Trp Gly Leu Arg
225                 230                 235                 240

ttc tat gtg tct gga cat cca ggc gta cag ttc acc att cgc tta aaa 768
Phe Tyr Val Ser Gly His Pro Gly Val Gln Phe Thr Ile Arg Leu Lys
                245                 250                 255

atc acc aac atg cca gct gtg gca gta ggt cct gac ctc gtc ctt gtg 816
Ile Thr Asn Met Pro Ala Val Ala Val Gly Pro Asp Leu Val Leu Val
            260                 265                 270

gaa caa gga cct cct aga acg tcc ctc gct ctc cca cct cct ctt ccc 864
Glu Gln Gly Pro Pro Arg Thr Ser Leu Ala Leu Pro Pro Pro Leu Pro
        275                 280                 285

cca agg gaa gcg cca ccg cca tct ctc ccc gac tct aac tcc aca gcc 912

Pro Arg Glu Ala Pro Pro Pro Ser Leu Pro Asp Ser Asn Ser Thr Ala
    290                 295                 300

ctg gcg act agt gca caa act ccc acg gtg aga aaa aca att gtt acc      960
Leu Ala Thr Ser Ala Gln Thr Pro Thr Val Arg Lys Thr Ile Val Thr
305                 310                 315                 320

cta aac act ccg cct ccc acc aca ggc gac aga ctt ttt gat ctt gtg     1008
Leu Asn Thr Pro Pro Pro Thr Thr Gly Asp Arg Leu Phe Asp Leu Val
                325                 330                 335

cag ggg gcc ttc cta acc tta aat gct acc aac cca ggg gcc act gag     1056
Gln Gly Ala Phe Leu Thr Leu Asn Ala Thr Asn Pro Gly Ala Thr Glu
            340                 345                 350

tct tgc tgg ctt tgt ttg gcc atg ggc ccc cct tat tat gaa gca ata     1104
Ser Cys Trp Leu Cys Leu Ala Met Gly Pro Pro Tyr Tyr Glu Ala Ile
        355                 360                 365

gcc tca tca gga gag gtc gcc tac tcc acc gac ctt gac cgg tgc cgc     1152
Ala Ser Ser Gly Glu Val Ala Tyr Ser Thr Asp Leu Asp Arg Cys Arg
    370                 375                 380

tgg ggg acc caa gga aag ctc acc ctc act gag gtc tca gga cac ggg     1200
Trp Gly Thr Gln Gly Lys Leu Thr Leu Thr Glu Val Ser Gly His Gly
385                 390                 395                 400

ttg tgc ata gga aag gtg ccc ttt acc cat cag cat ctc tgc aat cag     1248
Leu Cys Ile Gly Lys Val Pro Phe Thr His Gln His Leu Cys Asn Gln
                405                 410                 415

acc cta tcc atc aat tcc tcc gga gac cat cag tat ctg ctc ccc tcc     1296
Thr Leu Ser Ile Asn Ser Ser Gly Asp His Gln Tyr Leu Leu Pro Ser
            420                 425                 430

aac cat agc tgg tgg gct tgc agc act ggc ctc acc cct tgc ctc tcc     1344
Asn His Ser Trp Trp Ala Cys Ser Thr Gly Leu Thr Pro Cys Leu Ser
        435                 440                 445

acc tca gtt ttt aat cag act aga gat ttc tgt atc cag gtc cag ctg     1392
Thr Ser Val Phe Asn Gln Thr Arg Asp Phe Cys Ile Gln Val Gln Leu
    450                 455                 460

att cct cgc atc tat tac tat cct gaa gaa gtt ttg tta cag gcc tat     1440
Ile Pro Arg Ile Tyr Tyr Tyr Pro Glu Glu Val Leu Leu Gln Ala Tyr
465                 470                 475                 480

gac aat tct cac ccc agg act aaa aga gag gct gtc tca ctt acc cta     1488
Asp Asn Ser His Pro Arg Thr Lys Arg Glu Ala Val Ser Leu Thr Leu
                485                 490                 495

gct gtt tta ctg ggg ttg gga atc acg gcg gga ata ggt act ggt tca     1536
Ala Val Leu Leu Gly Leu Gly Ile Thr Ala Gly Ile Gly Thr Gly Ser
            500                 505                 510

act gcc tta att aaa gga cct ata gac ctc cag caa ggc ctg aca agc     1584
Thr Ala Leu Ile Lys Gly Pro Ile Asp Leu Gln Gln Gly Leu Thr Ser
        515                 520                 525

ctc cag atc gcc ata gat gct gac ctc cgg gcc ctc caa gac tca gtc 1632
Leu Gln Ile Ala Ile Asp Ala Asp Leu Arg Ala Leu Gln Asp Ser Val
    530                 535                 540

agc aag tta gag gac tca ctg act tcc ctg tcc gag gta gtg ctc caa 1680
Ser Lys Leu Glu Asp Ser Leu Thr Ser Leu Ser Glu Val Val Leu Gln
545                 550                 555                 560

aat agg aga ggc ctt gac ttg ctg ttt cta aaa gaa ggt ggc ctc tgt 1728
Asn Arg Arg Gly Leu Asp Leu Leu Phe Leu Lys Glu Gly Gly Leu Cys
                565                 570                 575

gcg gcc cta aag gaa gag tgc tgt ttt tac ata gac cac tca ggt gca 1776
Ala Ala Leu Lys Glu Glu Cys Cys Phe Tyr Ile Asp His Ser Gly Ala
            580                 585                 590

gta cgg gac tcc atg aaa aaa ctc aaa gaa aaa ctg gat aaa aga cag 1824
Val Arg Asp Ser Met Lys Lys Leu Lys Glu Lys Leu Asp Lys Arg Gln
        595                 600                 605

tta gag cgc cag aaa agc caa aac tgg tat gaa gga tgg ttc aat aac 1872
Leu Glu Arg Gln Lys Ser Gln Asn Trp Tyr Glu Gly Trp Phe Asn Asn
    610                 615                 620

tcc cct tgg ttc act acc ctg cta tca acc atc gct ggg ccc cta tta 1920
Ser Pro Trp Phe Thr Thr Leu Leu Ser Thr Ile Ala Gly Pro Leu Leu
625                 630                 635                 640

ctc ctc ctt ctg ttg ctc atc ctc ggg cca tgc atc atc aat aag tta 1968
Leu Leu Leu Leu Leu Leu Ile Leu Gly Pro Cys Ile Ile Asn Lys Leu
                645                 650                 655

gtt caa ttc atc aat gat agg ata agt gca tgt taa aattctggtc 2014
Val Gln Phe Ile Asn Asp Arg Ile Ser Ala Cys
            660                 665

cttagacaaa atatcaggcc ctagagaacg aaggtaacct ttaattttgc tctaagatta 2074

gagctattca caagagaaat gggggaatga aagaagtgtt tttttttagc caact 2129

<210> 6
<211> 2001
<212> ADN
<213> Séquence artificielle

<220>
<223> Description de la séquence artificielle: Enveloppe
Amphotrope 4070A

<220>
<221> CDS
<222> (1)..(2001)

<400> 6

ggc cga cac cca gag tgg acc atc ctc tgg acg gac atg gcg cgt tca    48
Gly Arg His Pro Glu Trp Thr Ile Leu Trp Thr Asp Met Ala Arg Ser
1               5                   10                  15

acg ctc tca aaa ccc cct caa gat aag att aac ccg tgg aag ccc tta    96
Thr Leu Ser Lys Pro Pro Gln Asp Lys Ile Asn Pro Trp Lys Pro Leu
            20                  25                  30

ata gtc atg gga gtc ctg tta gga gta ggg atg gca gag agc ccc cat    144
Ile Val Met Gly Val Leu Leu Gly Val Gly Met Ala Glu Ser Pro His
        35                  40                  45

cag gtc ttt aat gta acc tgg aga gtc acc aac ctg atg act ggg cgt    192
Gln Val Phe Asn Val Thr Trp Arg Val Thr Asn Leu Met Thr Gly Arg
    50                  55                  60

acc gcc aat gcc acc tcc ctc ctg gga act gta caa gat gcc ttc cca    240
Thr Ala Asn Ala Thr Ser Leu Leu Gly Thr Val Gln Asp Ala Phe Pro
65                  70                  75                  80

aaa tta tat ttt gat cta tgt gat ctg gtc gga gag gag tgg gac cct    288
Lys Leu Tyr Phe Asp Leu Cys Asp Leu Val Gly Glu Glu Trp Asp Pro
                85                  90                  95

tca gac cag gaa ccg tat gtc ggg tat ggc tgc aag tac ccc gca ggg    336
Ser Asp Gln Glu Pro Tyr Val Gly Tyr Gly Cys Lys Tyr Pro Ala Gly
            100                 105                 110

aga cag cgg acc cgg act ttt gac ttt tac gtg tgc cct ggg cat acc    384
Arg Gln Arg Thr Arg Thr Phe Asp Phe Tyr Val Cys Pro Gly His Thr
        115                 120                 125

gta aag tcg ggg tgt ggg gga cca gga gag ggc tac tgt ggt aaa tgg    432
Val Lys Ser Gly Cys Gly Gly Pro Gly Glu Gly Tyr Cys Gly Lys Trp
    130                 135                 140

ggg tgt gaa acc acc gga cag gct tac tgg aag ccc aca tca tcg tgg    480
Gly Cys Glu Thr Thr Gly Gln Ala Tyr Trp Lys Pro Thr Ser Ser Trp
145                 150                 155                 160

gac cta atc tcc ctt aag cgc ggt aac acc ccc tgg gac acg gga tgc    528
Asp Leu Ile Ser Leu Lys Arg Gly Asn Thr Pro Trp Asp Thr Gly Cys
                165                 170                 175

tct aaa gtt gcc tgt ggc ccc tgc tac gac ctc tcc aaa gta tcc aat    576
Ser Lys Val Ala Cys Gly Pro Cys Tyr Asp Leu Ser Lys Val Ser Asn
            180                 185                 190

tcc ttc caa ggg gct act cga ggg ggc aga tgc aac cct cta gtc cta    624
Ser Phe Gln Gly Ala Thr Arg Gly Gly Arg Cys Asn Pro Leu Val Leu
        195                 200                 205

gaa ttc act gat gca gga aaa aag gct aac tgg gac ggg ccc aaa tcg    672
Glu Phe Thr Asp Ala Gly Lys Lys Ala Asn Trp Asp Gly Pro Lys Ser
    210                 215                 220

tgg gga ctg aga ctg tac cgg aca gga aca gat cct att acc atg ttc    720
Trp Gly Leu Arg Leu Tyr Arg Thr Gly Thr Asp Pro Ile Thr Met Phe

225                 230                 235                 240

tcc ctg acc cgg cag gtc ctt aat gtg gga ccc cga gtc ccc ata ggg     768
Ser Leu Thr Arg Gln Val Leu Asn Val Gly Pro Arg Val Pro Ile Gly
                245                 250                 255

ccc aac cca gta tta ccc gac caa aga ctc cct tcc tca cca ata gag     816
Pro Asn Pro Val Leu Pro Asp Gln Arg Leu Pro Ser Ser Pro Ile Glu
            260                 265                 270

att gta ccg gct cca cag cca cct agc ccc ctc aat acc agt tac ccc     864
Ile Val Pro Ala Pro Gln Pro Pro Ser Pro Leu Asn Thr Ser Tyr Pro
        275                 280                 285

cct tcc act acc agt aca ccc tca acc tcc cct aca agt cca agt gtc     912
Pro Ser Thr Thr Ser Thr Pro Ser Thr Ser Pro Thr Ser Pro Ser Val
    290                 295                 300

cca cag cca ccc cca gga act gga gat aga cta cta gct cta gtc aaa     960
Pro Gln Pro Pro Pro Gly Thr Gly Asp Arg Leu Leu Ala Leu Val Lys
305                 310                 315                 320

gga gcc tat cag gcg ctt aac ctc acc aat ccc gac aag acc caa gaa    1008
Gly Ala Tyr Gln Ala Leu Asn Leu Thr Asn Pro Asp Lys Thr Gln Glu
                325                 330                 335

tgt tgg ctg tgc tta gtg tcg gga cct cct tat tac gaa gga gta gcg    1056
Cys Trp Leu Cys Leu Val Ser Gly Pro Pro Tyr Tyr Glu Gly Val Ala
            340                 345                 350

gtc gtg ggc act tat acc aat cat tcc acc gct ccg gcc aac tgt acg    1104
Val Val Gly Thr Tyr Thr Asn His Ser Thr Ala Pro Ala Asn Cys Thr
        355                 360                 365

gcc act tcc caa cat aag ctt acc cta tct gaa gtg aca gga cag ggc    1152
Ala Thr Ser Gln His Lys Leu Thr Leu Ser Glu Val Thr Gly Gln Gly
    370                 375                 380

cta tgc atg ggg gca gta cct aaa act cac cag gcc tta tgt aac acc    1200
Leu Cys Met Gly Ala Val Pro Lys Thr His Gln Ala Leu Cys Asn Thr
385                 390                 395                 400

acc caa agc gcc ggc tca gga tcc tac tac ctt gca gca ccc gcc gga    1248
Thr Gln Ser Ala Gly Ser Gly Ser Tyr Tyr Leu Ala Ala Pro Ala Gly
                405                 410                 415

aca atg tgg gct tgc agc act gga ttg act ccc tgc ttg tcc acc acg    1296
Thr Met Trp Ala Cys Ser Thr Gly Leu Thr Pro Cys Leu Ser Thr Thr
            420                 425                 430

gtg ctc aat cta acc aca gat tat tgt gta tta gtt gaa ctc tgg ccc    1344
Val Leu Asn Leu Thr Thr Asp Tyr Cys Val Leu Val Glu Leu Trp Pro
        435                 440                 445

aga gta att tac cac tcc ccc gat tat atg tat ggt cag ctt gaa cag    1392
Arg Val Ile Tyr His Ser Pro Asp Tyr Met Tyr Gly Gln Leu Glu Gln
    450                 455                 460

cgt acc aaa tat aaa aga gag cca gta tca ttg acc ctg gcc ctt cta     1440
Arg Thr Lys Tyr Lys Arg Glu Pro Val Ser Leu Thr Leu Ala Leu Leu
465                 470                 475                 480

cta gga gga tta acc atg gga ggg att gca gct gga ata ggg acg ggg     1488
Leu Gly Gly Leu Thr Met Gly Gly Ile Ala Ala Gly Ile Gly Thr Gly
                485                 490                 495

acc act gcc tta att aaa acc cag cag ttt gag cag ctt cat gcc gct     1536
Thr Thr Ala Leu Ile Lys Thr Gln Gln Phe Glu Gln Leu His Ala Ala
            500                 505                 510

atc cag aca gac ctc aac gaa gtc gaa aag tca att acc aac cta gaa     1584
Ile Gln Thr Asp Leu Asn Glu Val Glu Lys Ser Ile Thr Asn Leu Glu
        515                 520                 525

aag tca ctg acc tcg ttg tct gaa gta gtc cta cag aac cgc aga ggc     1632
Lys Ser Leu Thr Ser Leu Ser Glu Val Val Leu Gln Asn Arg Arg Gly
    530                 535                 540

cta gat ttg cta ttc cta aag gag gga ggt ctc tgc gca gcc cta aaa     1680
Leu Asp Leu Leu Phe Leu Lys Glu Gly Gly Leu Cys Ala Ala Leu Lys
545                 550                 555                 560

gaa gaa tgt tgt ttt tat gca gac cac acg ggg cta gtg aga gac agc     1728
Glu Glu Cys Cys Phe Tyr Ala Asp His Thr Gly Leu Val Arg Asp Ser
                565                 570                 575

atg gcc aaa tta aga gaa agg ctt aat cag aga caa aaa cta ttt gag     1776
Met Ala Lys Leu Arg Glu Arg Leu Asn Gln Arg Gln Lys Leu Phe Glu
            580                 585                 590

aca ggc caa gga tgg ttc gaa ggg ctg ttt aat aga tcc ccc tgg ttt     1824
Thr Gly Gln Gly Trp Phe Glu Gly Leu Phe Asn Arg Ser Pro Trp Phe
        595                 600                 605

acc acc tta atc tcc acc atc atg gga cct cta ata gta ctc tta ctg     1872
Thr Thr Leu Ile Ser Thr Ile Met Gly Pro Leu Ile Val Leu Leu Leu
    610                 615                 620

atc tta ctc ttt gga cct tgc att ctc aat cga ttg gtc caa ttt gtt     1920
Ile Leu Leu Phe Gly Pro Cys Ile Leu Asn Arg Leu Val Gln Phe Val
625                 630                 635                 640

aaa gac agg atc tca gtg gtc cag gct ctg gtt ttg act cag caa tat     1968
Lys Asp Arg Ile Ser Val Val Gln Ala Leu Val Leu Thr Gln Gln Tyr
                645                 650                 655

cac cag cta aaa ccc ata gag tac gag cca tga                         2001
His Gln Leu Lys Pro Ile Glu Tyr Glu Pro
            660                 665

<210> 7
<211> 32
<212> ADN

<213> Séquence artificielle

<220>
<223> Description de la séquence artificielle:
      Oligonucleotide OUGalv5'Nsi

<400> 7
gagcgccaga aaagccaaaa ctggtatgaa gg                                32


<210> 8
<211> 50
<212> ADN
<213> Séquence artificielle

<220>
<223> Description de la séquence artificielle:
      Oligonucleotide OLCT 1Cla

<400> 8
gatcatcgat tatttattta cacttctttc attcccccat ttctcttgtg             50


<210> 9
<211> 48
<212> ADN
<213> Séquence artificielle

<220>
<223> Description de la séquence artificielle:
      Oligonucleotide OLCT 2Cla

<400> 9
gatcatcgat tatttattta aaggttacct tcgttctcta gggcctga               48


<210> 10
<211> 51
<212> ADN
<213> Séquence artificielle

<220>
<223> Description de la séquence artificielle:
      Oligonucleotide OLCT3Cla

<400> 10
gatcatcgat tatttattta tagggcctga tattttgtct aaggaccaga a           51


<210> 11
<211> 51
<212> ADN
<213> Séquence artificielle

<220>
<223> Description de la séquence artificielle:
      Oligonucleotide OLCT4Cla

<400> 11
gatcatcgat tatttattta acatgcactt atcctatcat tgatgaattg a          51


<210> 12
<211> 40
<212> ADN
<213> Séquence artificielle

<220>
<223> Description de la séquence artificielle:
      Oligonucleotide 5' Nhe1

<400> 12
gctagcatgg tattgctgcc tgggtccatg cttctcacct                       40


<210> 13
<211> 31
<212> ADN
<213> Séquence artificielle

<220>
<223> Description de la séquence artificielle:
      Oligonucleotide 3' Xba1

<400> 13
tctagattaa catgcactta tcctatcatt g                                31


<210> 14
<211> 38
<212> ADN
<213> Séquence artificielle

<220>
<223> Description de la séquence artificielle:
      Oligonucleotide 3' Xba1

<400> 14
tctagaagtt ggctaaaaaa aaacacttct ttcattcc                         38

## Description

La présente invention concerne de nouvelles protéines et compositions utilisables pour la production de virus, notamment rétrovirus recombinants. Elle concerne également des acides nucléiques, vecteurs et cellules modifiées, ainsi que leurs utilisations. L'invention est plus particulièrement relative à de nouvelles protéines dérivées du virus GALV et leurs utilisations.

Les rétrovirus recombinants sont utilisés dans de nombreuses applications de la biologie, à la fois sur la plan expérimental, diagnostic, vaccinal et thérapeutique. Ainsi, les rétrovirus sont utilisés comme vecteur pour le transfert d'acides nucléiques dans les cellules, essentiellement les cellules en division, in vitro, ex vivo ou in vivo. In vitro, les rétrovirus permettent ainsi, par exemple, d'insérer de manière stable un acide nucléique d'intérêt dans le génome d'une cellule, de manière à produire une protéine ou un peptide recombinant, à étudier la régulation d'un gène, à étudier des composés antiviraux, etc. In vivo et ex vivo, les rétrovirus ont été utilisés dans des protocoles cliniques de transfert de gènes à visée thérapeutique et/ou vaccinale. Pour revue, voir notamment Human Gene Therapy, Décembre 1997. Les rétrovirus sont également utilisables pour le transfert de gènes chez des mammifères non-humains, par exemple dans des buts thérapeutiques, vaccinaux, ou expérimentaux (modèles de pathologies, étude de biodisponibilité, etc.).

En raison de leur large spectre d'applications évoqué ci-avant, il est important de pouvoir disposer de méthodes efficaces et présentant un caractère sécuritaire élevé pour produire des rétrovirus recombinants ayant de bonnes capacités de transfert de gènes.

A cet égard, les vecteurs rétroviraux recombinants utilisés comportent généralement, dans une capside rétrovirale, un génome rétroviral défectif. Plus précisément, le génome rétroviral défectif est un vecteur rétroviral recombinant comprenant généralement les séquences cis indispensables à la production et l'encapsidation du génome, c'est-à-dire :
- les séquences "Long Terminal Repeat" (LTR), localisées à chaque extrémité du génome rétroviral. Ces séquences servent notamment d'origine de transcription et de promoteur transcriptionnel. Les séquences LTR se composent plus précisément d'éléments désignés U3, R et U5 ;
- une séquence d'encapsidation (Psi ou ψ), impliquée dans l'encapsidation du génome rétroviral dans l'enveloppe protéique. La séquence d'encapsidation peut par ailleurs comporter une région étendue à certains éléments du gène gag, qui ont été décrits comme améliorant l'efficacité d'encapsidation ;
- la région PB, également impliquée dans la réplication virale.

En général, le génome rétroviral défectif est en revanche défectif pour tout ou partie des gènes viraux gag, pol et/ou env. L'absence de tout ou partie des gènes viraux rend en effet le virus défectif, c'est-à-dire incapable de réplication autonome dans une cellule. Plus préférentiellement, l'ensemble des gènes viraux gag, pol et env sont délétés. Le génome rétroviral défectif comprend par ailleurs, en substitution des séquences virales délétées, tout acide nucléique d'intérêt dont le transfert dans une cellule est recherché.

Pour la construction de rétrovirus défectifs, le génome rétroviral peut provenir de différents types de rétrovirus, tels que des virus écotropes et/ou amphotropes. Il peut s'agir notamment de rétrovirus appartenant à la famille des oncovirus, des lentivirus ou des spumavirus. Dans la famille des oncovirus, on peut citer notamment les oncovirus lents, non porteur d'oncogène, tels que par exemple MoMLV, ALV, BLV, ou MMTV et les oncovirus rapides, tels que RSV par exemple. Dans la famille des lentivirus, on peut citer par exemple HIV, SIV, FIV, BIV ou CAEV. Un type de rétrovirus particulièrement utilisé est le virus de Moloney, notamment murin (MoMLV).

Pour la production des rétrovirus recombinants, le vecteur rétroviral (génome rétroviral défectif ou toute construction le comprenant) est généralement introduit dans une cellule dite de "packaging", exprimant de manière stable les fonctions de complémentation déficientes du génome recombinant (i.e., gag, pol et/ou env). De telles lignées sont par exemples les lignées PSICRIP (Danos et Mulligan, PNAS 85 (1988) p. 6460), PA317 (Miller et Buttimore, Mol. Cell. Biol. 6 (1986) p. 2895), ou GP + Env AM12 (Markowitz et al. Virology 167 (1988) p. 400). D'autres lignées ont par exemple été décrites dans les publications EP 243 204, WO89/07150, WO90/02806, US5,766,945, EP476,953, WO93/04167 et WO93/10218. En outre, d'autres approches ont été envisagées pour la production de rétrovirus recombinants, reposant sur la cotransfection transitoire de lignées cellulaires avec des virus (ou plasmides) helper et le vecteur rétroviral recombinant (voir par exemple W094/29438)

L'une des particularités des rétrovirus réside dans leur sélectivité pour les cellules en division. Ainsi, dans les conditions naturelles, les retrovirus ne sont pas capables d'infecter efficacement les cellules quiescentes. Par ailleurs, le tropisme des rétrovirus est essentiellement déterminé par la protéine d'enveloppe présente dans la capside. Ainsi, il est possible de construire des rétrovirus écotropes (c'est-à-dire dont le tropisme est restreint à l'espèce dont provient l'enveloppe) ou amphotropes (c'est-à-dire dont le tropisme croise les barrières d'espèces). Des enveloppes particulièrement utilisées pour la construction de rétrovirus sont par exemple les enveloppes des virus 4070A, 10A1, VSV, VIH, du virus de la rage, du virus GALV, ou également des enveloppes cellulaires, codant par exemple pour une protéine membranaire permettant le ciblage de la particule virale sur un ligand spécifique, notamment pour un récepteur de type CD4.

L'enveloppe du virus GALV présente un intérêt particulier. En effet, il a été décrit dans la littérature que le virus de la leucémie du gibbon (GALV ; "Gibbon Ape Leukemia Virus"), notamment son enveloppe, avait un tropisme particulièrement important pour les cellules humaines, notamment hématopoiétiques (cellules souches, lymphocytes, dendritiques, etc.). Il a donc été envisagé, dans l'art antérieur, d'utiliser des rétrovirus recombinants pseudotypés avec l'enveloppe GALV, c'est-à-dire des rétrovirus recombinants de type Moloney par exemple, comprenant une enveloppe GALV, afin d'améliorer les propriétés d'infection des cellules humaines, notamment hématopoiétiques (Bunnel et al., PNAS 92 (1995) 7739). Certains résultats particulièrement encourageants ont été obtenus avec ces rétrovirus.

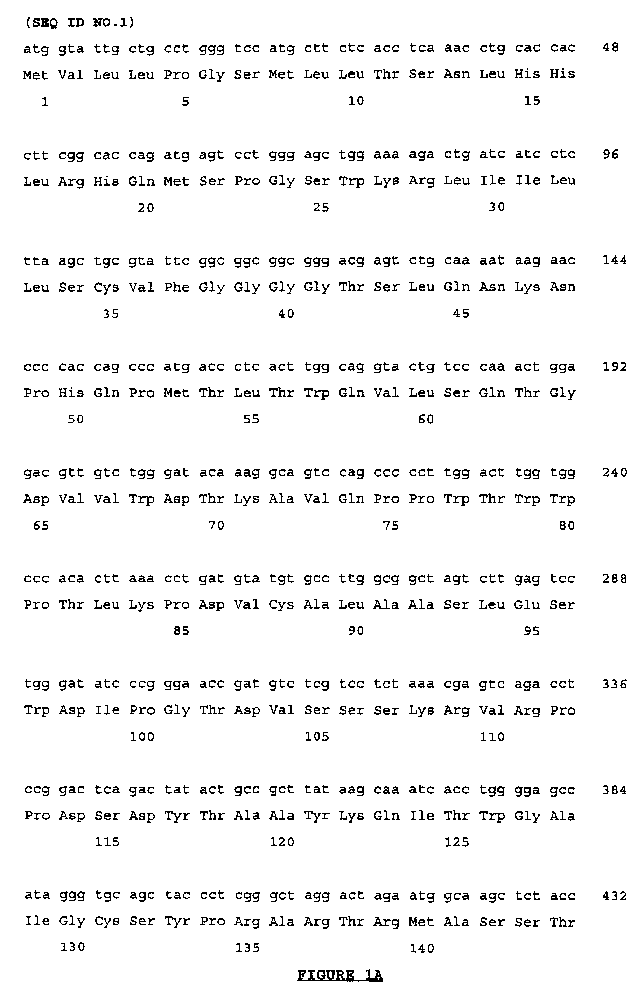

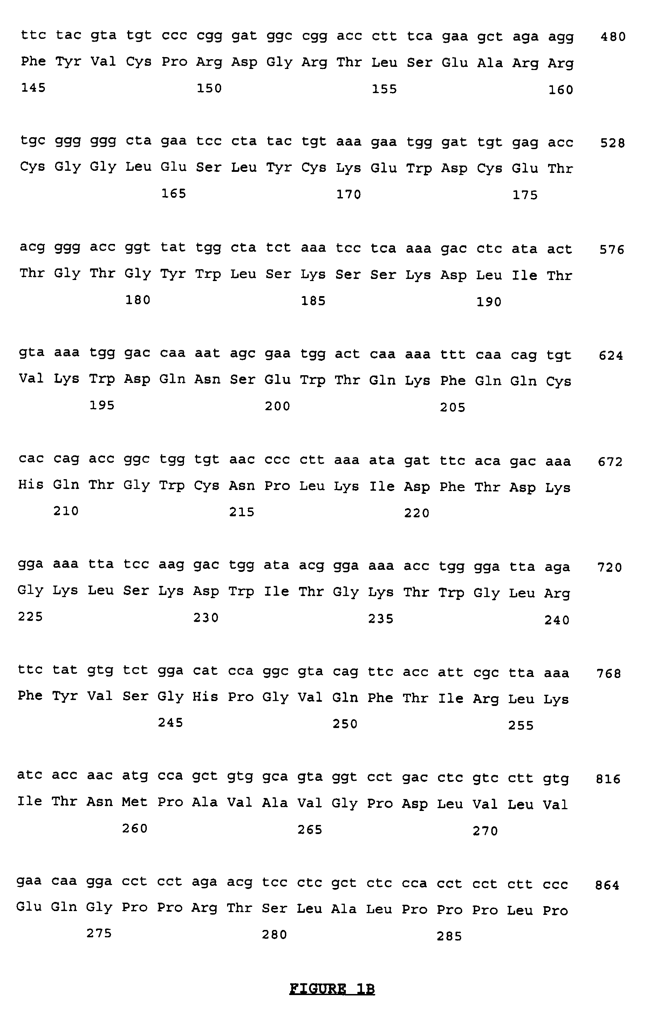

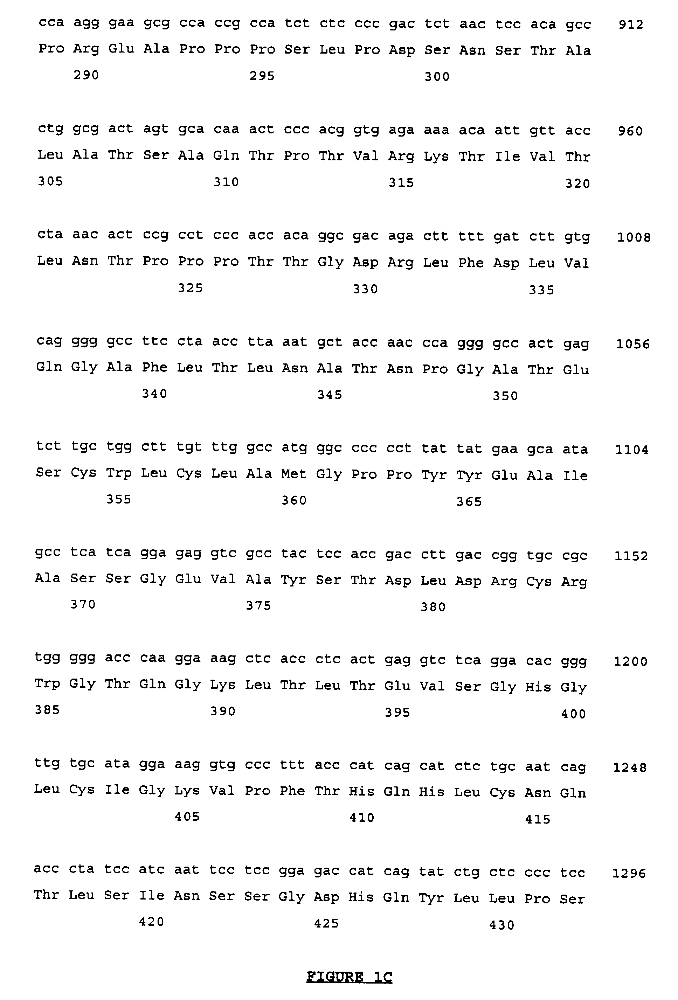

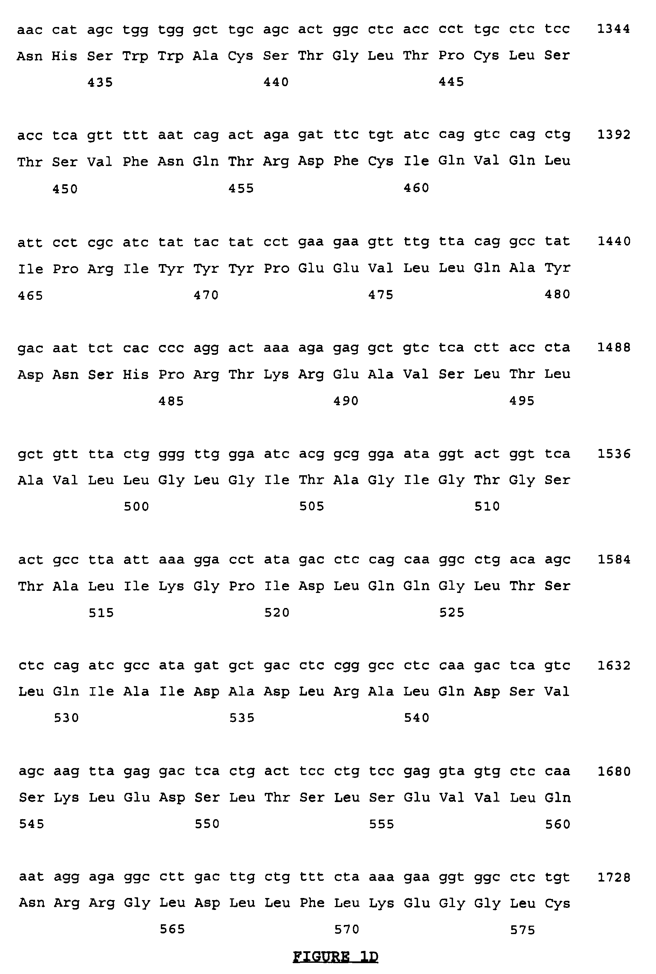

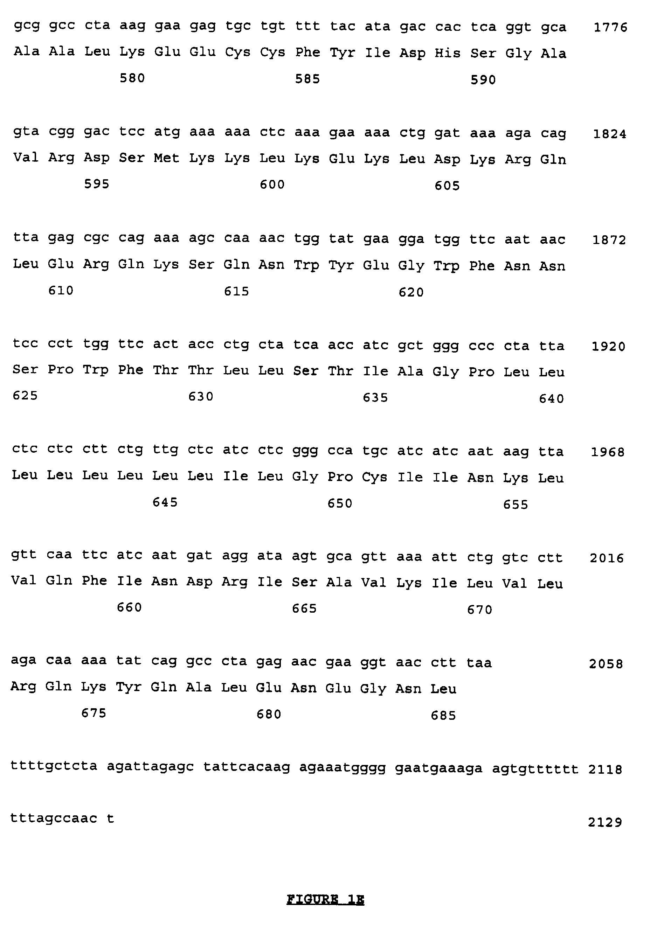

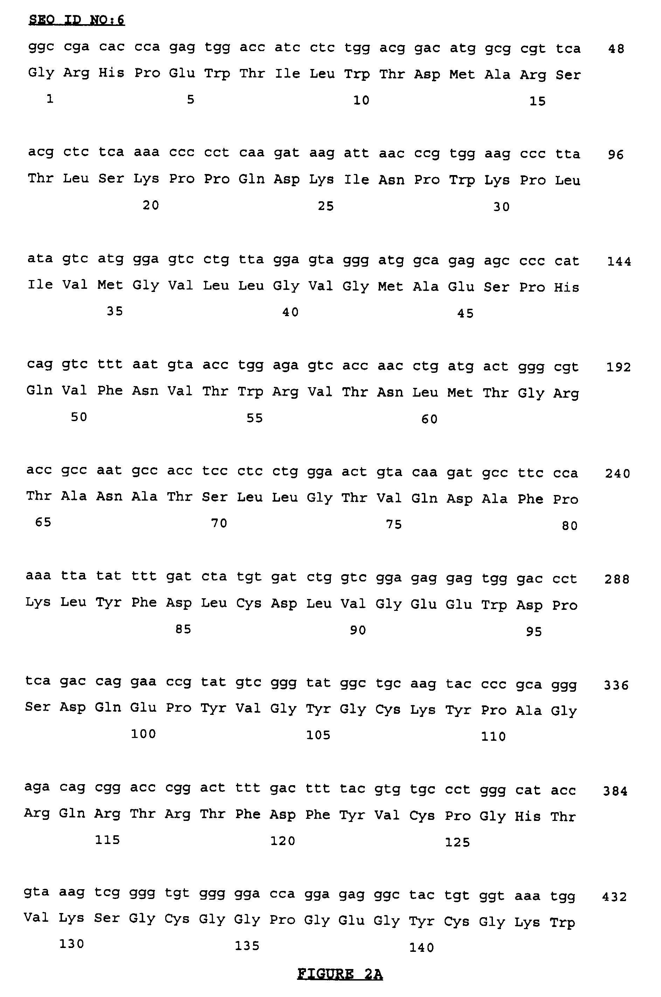

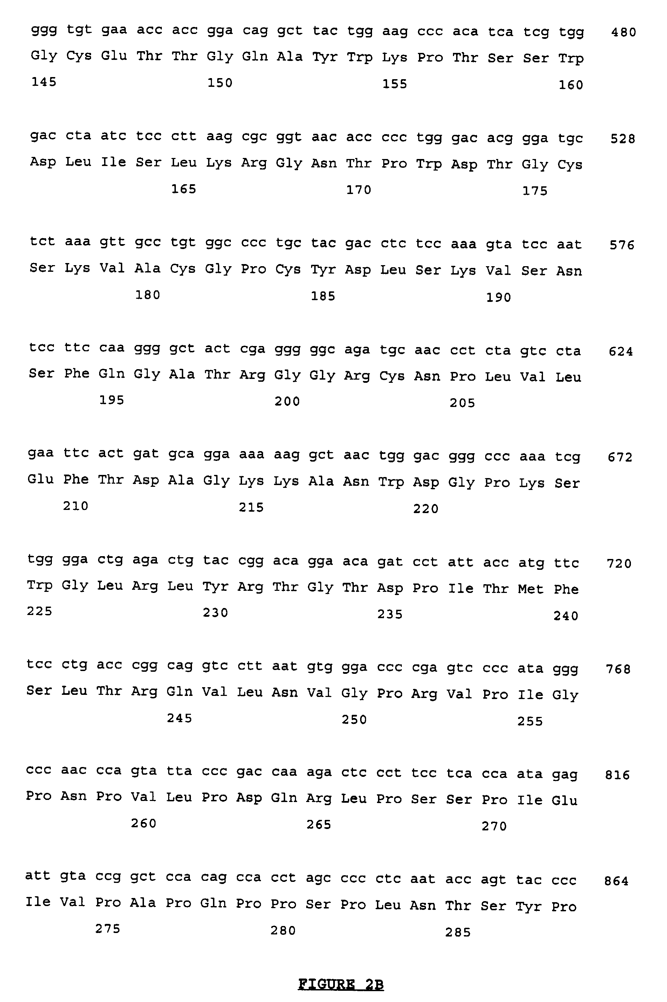

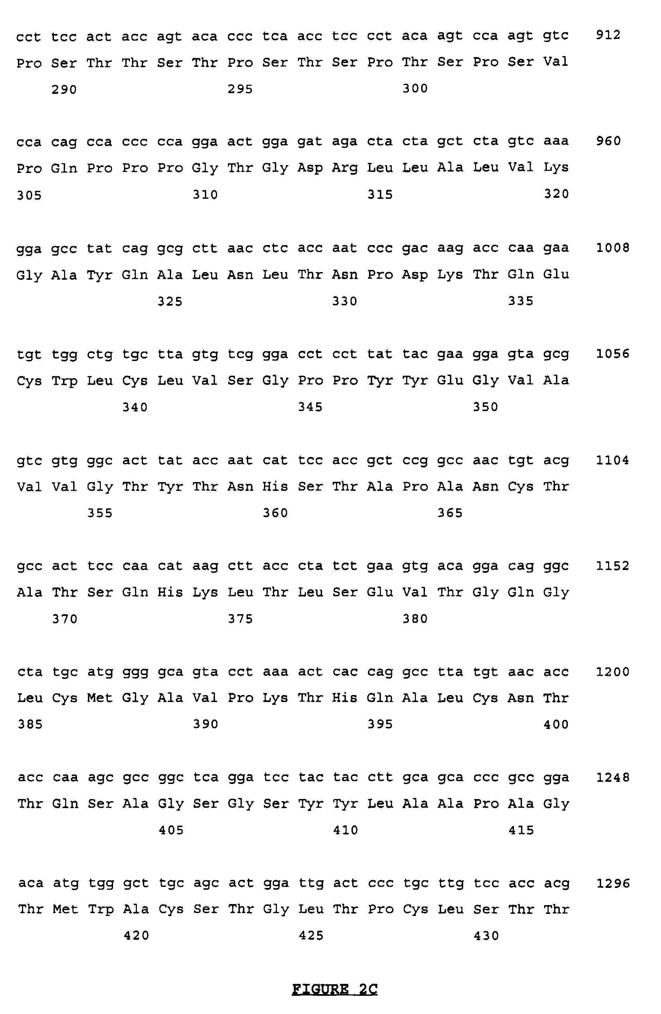

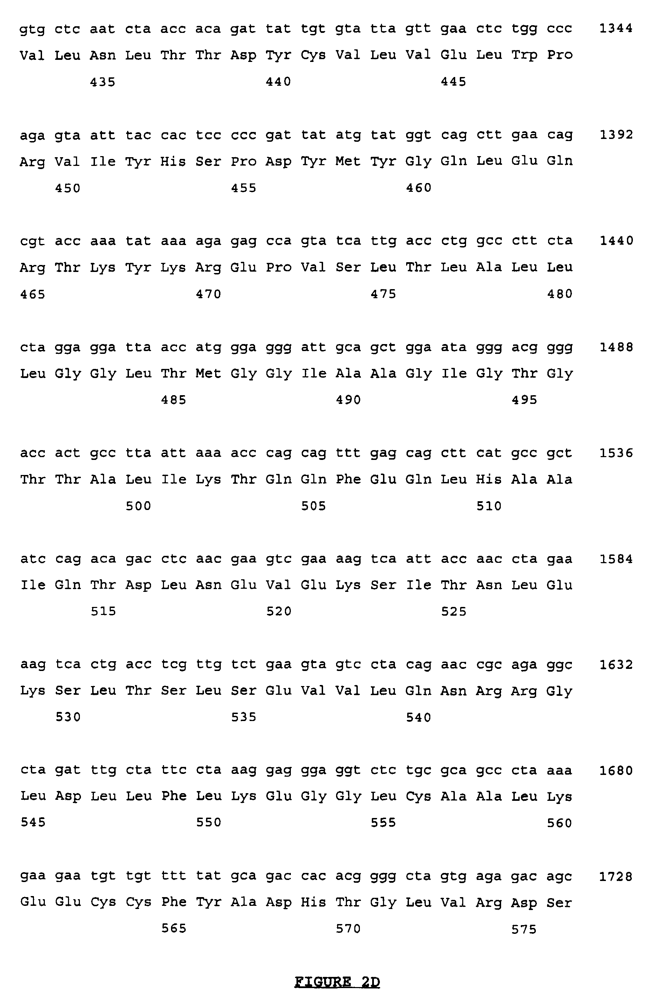

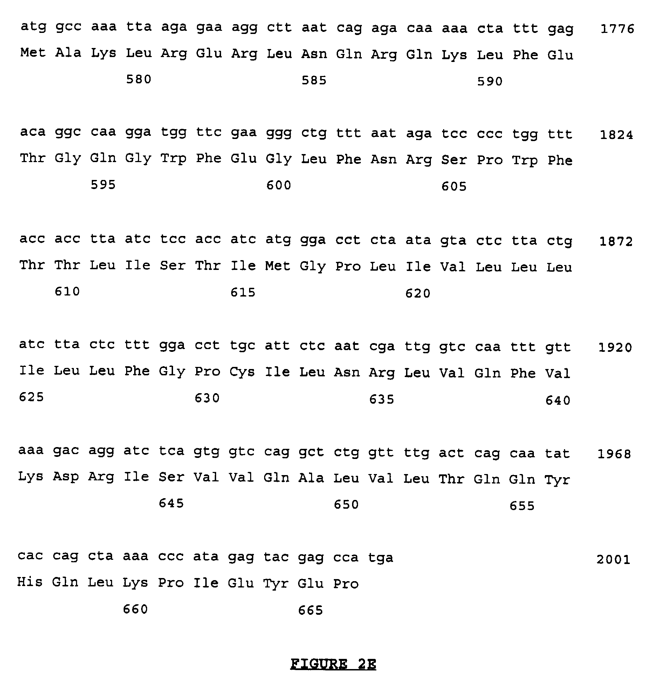

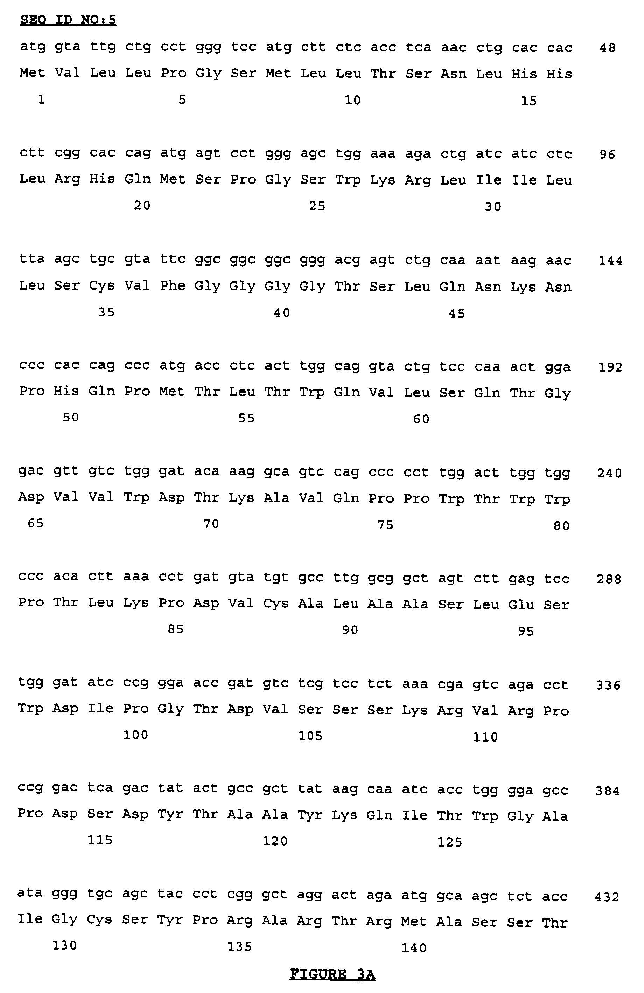

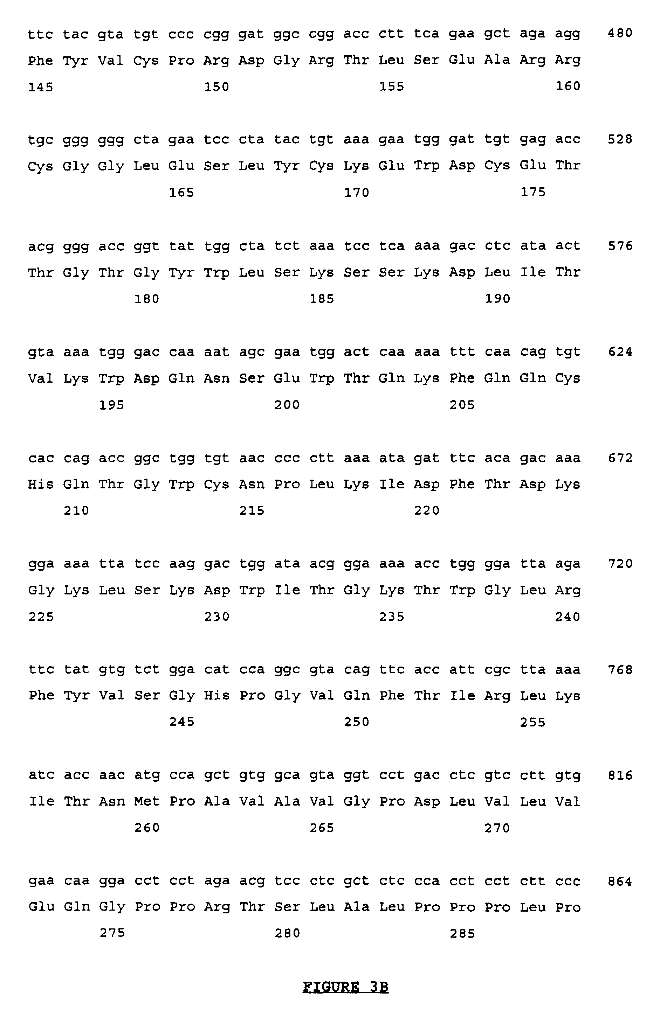

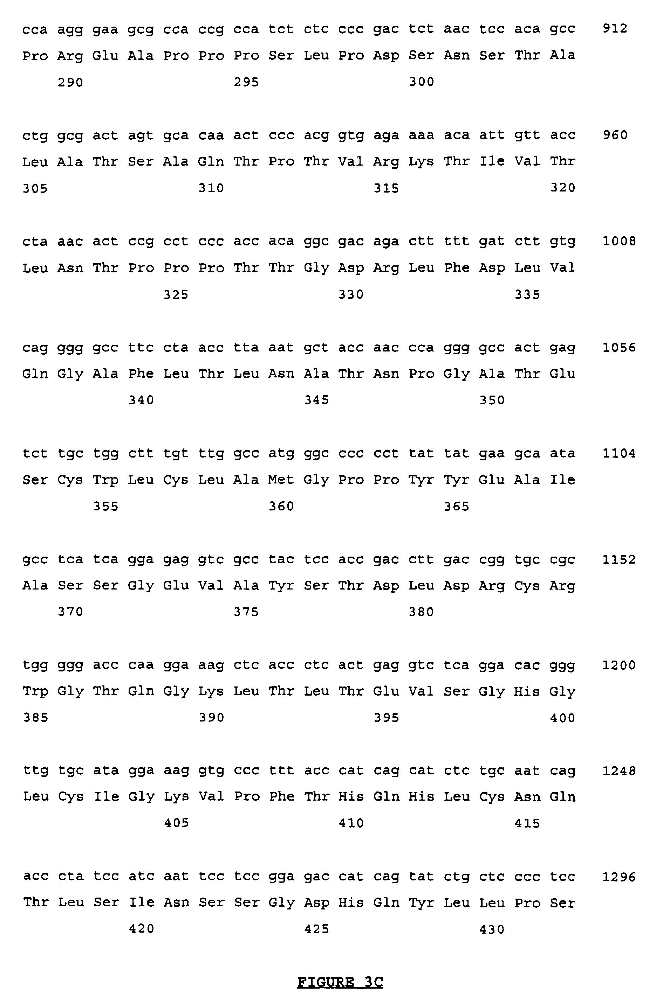

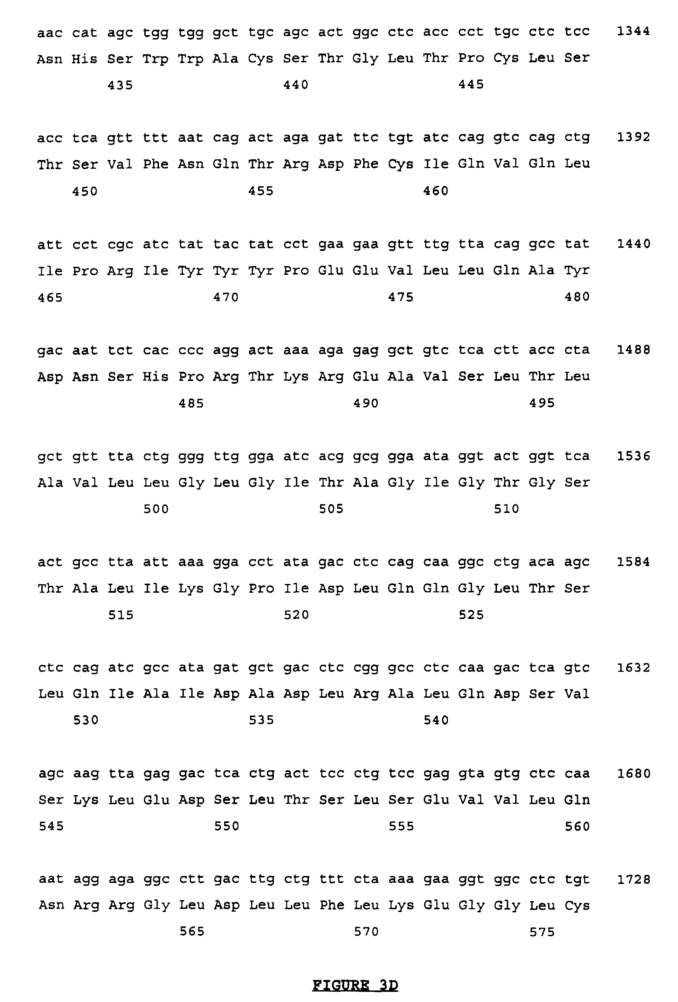

Néanmoins, les demandeurs ont maintenant montré que la protéine d'enveloppe GALV telle que décrite dans l'art antérieur, ne pouvait être utilisée efficacement, pour la production de rétrovirus recombinants, que dans certains types limités de cellules de packaging. Ainsi, alors que cette protéine GALV peut être utilisée pour produire des rétrovirus dans les cellules de fibroblastes murins (NIH 3T3) par exemple, cette même enveloppe ne peut être mise en oeuvre efficacement dans des lignées de packaging établies à partir de cellules humaines, notamment. En particulier, les inventeurs de la présente demande ont mis en évidence que la protéine env du virus GALV tel que décrit par Delassus et al. (Virology 173 (1989) 205) (voir résidus d'acides aminés 1 à 667 tels que représentés sur la figure 3 ou SEQ ID NO:5), exprimée dans différents types cellulaires possédant un récepteur à l'enveloppe GALV, produit un effet fusogénique important, conduisant à une fusion des cellules en culture, empêchant toute production de rétrovirus recombinants.

La présente invention apporte à présent une solution à ce problème.

Ainsi, la présente invention décrit de nouvelles constructions permettant la production d'une enveloppe de type GALV fonctionnelle, non fusogénique dans les cellules. En particulier, la présente invention découle notamment de la mise en évidence d'une protéine d'enveloppe GALV non fusogénique, et de la construction de molécules chimériques, artificielles, tronquées et/ou étendues ayant des propriétés avantageuses.

La présente invention découle aussi de la mise en évidence, par les inventeurs, que la fusogénicité de protéines d'enveloppe peut être contrôlée par une région présente dans le domaine C-terminal de la protéine. A cet égard, l'invention décrit aussi de nouvelles protéines d'enveloppe rétrovirales (notamment de type GALV), modifiées (notamment dans la région C-terminale), présentant une activité fusogénique, et leur utilisation pour induire la destruction de cellules, in vitro, ex vivo ou in vivo.

Ainsi, dans un premier aspect, l'invention réside en particulier dans des constructions chimériques, tronquées et/ou étendues de l'enveloppe GALV, permettant de réduire, voire supprimer les propriétés fusogéniques de cette protéine dans les cellules possédant un récepteur de l'enveloppe GALV. Plus particulièrement, l'invention décrit des enveloppes chimériques de type GALV, dépourvues de fusogénicité, caractérisées en ce qu'elles comprennent un domaine C-terminal (ou cytoplasmique) hétérologue. L'invention décrit aussi une nouvelle protéine d'enveloppe GALV, caractérisée par un domaine C-terminal particulier, également dépourvue de fusogénicité. L'invention réside également dans des variants de ces enveloppes, les acides nucléiques et vecteurs correspondants, et leurs utilisations, notamment pour la production de particules rétrovirales recombinantes. L'invention est également relative aux cellules modifiées, notamment des cellules de packaging, comprenant les constructions génétiques de l'invention, ainsi qu'à des rétrovirus recombinants et des méthodes pour leur production.

Selon un autre aspect, la présente invention réside également dans l'utilisation des propriétés fusogéniques de protéines d'enveloppes pour induire la destruction de cellules. Ainsi, l'invention décrit également la construction d'enveloppes (rétrovirales) fusogènes modifiées, notamment dans leur région cytoplasmique. L'invention réside dans l'utilisation de telles protéines fusogènes comme molécules toxiques pour induire la destruction de cellules in vitro, ex vivo ou in vivo. L'invention décrit en outre un procédé de préparation de protéines d'enveloppes fusogéniques.

Comme indiqué ci-avant, la présente invention découle donc notamment de la mise en évidence que des constructions génétiques peuvent être préparées permettant la production d'enveloppes de type GALV sans effet fusogénique. Ainsi, les demandeurs ont maintenant mis en évidence la séquence d'une protéine d'enveloppe GALV dépourvue d'activité fusogène. Les séquences de cette protéine et de l'acide nucléique codant correspondant sont représentées sur la Figure 1 (SEQ ID NOS:1 et 2). Ainsi, par rapport à la séquence de la protéine d'enveloppe de GALV telle que décrite dans la littérature (SEQ ID NO:5), il apparait que cette séquence comprend plusieurs modifications dans la région C-terminale, et notamment le remplacement de la cystéine 667 par une valine, et l'ajout de 18 acides aminés C-terminaux supplémentaires. Ainsi, cette protéine d'enveloppe comporte une extrémité C-terminale particulière, de séquence Val-Lys-Ile-Leu-Val-Leu-Arg-Gln-Lys-Tyr-Gln-Ala-Leu-Glu-Asn-Glu-Gly-Asn-Leu (SEQ ID NO:3). Un objet de la présente invention réside donc dans une protéine d'enveloppe rétrovirale GALV, caractérisée en ce qu'elle comprend une extrémité C-terminale de séquence SEQ ID NO:3 ou un variant fonctionnel de celle-ci, c'est-à-dire une séquence modifiée, comme détaillé plus loin, sans induire de fusogénicité pour la protéine d'enveloppe. Plus particulièrement, l'invention concerne une protéine d'enveloppe rétrovirale GALV, caractérisée en ce qu'elle comprend un domaine intracytoplasmique de séquence Lys-Leu-Val-Gln-Phe-lle-Asn-Asp-Arg-lle-Ser-Ala-Val-Lys-Ile-Leu-Val-Leu-Arg-Gln-Lys-Tyr-Gln-Ala-Leu-Glu-Asn-Glu-Gly-Asn-Leu (SEQ ID NO:4) ou un variant fonctionnel de celle-ci. La présente invention montre maintenant qu'une protéine d'enveloppe GALV telle que définie ci-dessus n'est pas fusogénique, et peut donc être utilisée pour la construction de rétrovirus recombinants. L'invention concerne également tout anticorps dirigé contre la séquence SEQ ID NO:3 définie ci-dessus.

La séquence complète de la protéine d'enveloppe GALV est représentée sur la séquence SEQ ID NO: 2 (Figure 1). La phase codant pour la protéine d'enveloppe s'étend du nucléotide en position 1 jusqu'au nucléotide en position 2058 de la séquence SEQ ID NO:1. Le codon TAA aux positions 2056-2058 correspond au codon stop. Le nucléotide en position 1 correspond au nucléotide 5552 du génome du virus GALV (Delassus S. et al., Virology 173, 205-213 (1989)). L'enveloppe GALV complète codée par cette séquence comporte donc 685 acides aminés, et non 667 comme décrit par Delassus et al. Les résultats présentés dans les exemples indiquent que l'expression de cette protéine complète n'induit pas la fusion de cellules portant le récepteur Galv.

Par ailleurs, l'invention montre également que des protéines chimériques peuvent être construites, conservant le tropisme des enveloppes GALV, mais dépourvues de fusogénicité, par délétion de tout ou partie du domaine intracytoplasmique et substitution de celui-ci par un domaine intracyoplasmique hétérologue. L'invention décrit en effet la partie minimale du domaine C-terminal de l'enveloppe qu'il est nécessaire de conserver pour abolir l'effet fusogène des protéines d'enveloppe.

Ainsi, un objet de l'invention réside également dans une protéine d'enveloppe chimérique caractérisée en ce qu'elle comprend au moins une partie du domaine extracellulaire de l'enveloppe du virus GALV ou d'un variant fonctionnel de celui-ci.

L'invention a aussi pour objet toute protéine d'enveloppe chimérique comprenant, liés de manière fonctionnelle:
(i) une partie N-terminale comprenant le domaine extracellulaire de la protéine, choisie pour un tropisme désiré,
(ii) un domaine transmembranaire, et
(iii) une extrémité C-terminale comprenant le domaine (intra)cytoplasmique de la protéine, choisie de telle sorte que la protéine d'enveloppe chimérique ne soit pas fusogène dans les cellules.

Au sens de l'invention, le terme "chimérique" désigne une protéine comprenant deux éléments au moins ayant une origine distincte. Il peut s'agir par exemple de deux éléments provenant de protéines d'espèces différentes ou ayant des propriétés différentes. Généralement, le terme chimérique désigne une protéine hybride entre deux régions au moins de deux protéines d'enveloppe hétérologues ou entre une région d'une enveloppe et une région d'une protéine distincte, par exemple une partie cytoplasmique et éventuellement transmembranaire d'une protéine d'enveloppe et une partie N-terminale provenant de toute autre protéine capable de conférer à la molécule un tropisme donné (anticorps ou fragments ou dérivés, ligands, récepteur, etc.). La protéine chimérique peut être le résultat d'une association chimique, génétique ou biologique. Généralement, il s'agit d'un couplage génétique, donnant lieu à une protéine de fusion, par expression dans une cellule appropriée.

Les protéines d'enveloppe selon l'invention sont donc généralement composées de trois domaines fonctionnels principaux, un domaine extracellulaire, un domaine transmembranaire et un domaine cytoplasmique.

Comme indiqué ci-avant, dans un mode particulier de mise en oeuvre, les protéines chimériques de l'invention comprennent comme région N-terminale (i), tout ou partie du domaine extracellulaire de la protéine d'enveloppe de GALV ou d'un variant fonctionnel de celui-ci. De manière surprenante, les demandeurs ont en effet montré que le caractère fusogénique de GALV pouvait être supprimé (ou réduit), tout en conservant le domaine extracellulaire (et donc le tropisme avantageux) de cette enveloppe. Ce résultat est d'autant plus surprenant que le domaine extracellulaire, de par sa localisation (exposé à la surface des membranes des cellules qui le produisent, et également des particules rétrovirales), pouvait a priori être supposé jouer un rôle essentiel dans le phénomène de fusion. Les résultats présentés dans la présente demande de brevet montrent maintenant que des enveloppes chimériques de type GALV peuvent être construites, fonctionnelles, conservant le tropisme du GALV, et non fusogéniques. Ces constructions permettent donc de produire des rétrovirus recombinants dans des conditions avantageuses, comme il sera explicité plus loin.

Le domaine extracellulaire de la protéine d'enveloppe GALV selon l'invention correspond essentiellement aux résidus d'acides aminés 1 à 490 (correspondant aux nucléotides 1 à 1468) de la séquence SEQ ID NO: 1. Les résidus aux positions 491 à 655 (correspondant aux nucléotides 1469 à 1963) représentent le domaine transmembranaire de la protéine, et les résidus aux positions 656 à 685 (correspondant aux nucléotides 1964 à 2058) représentent le domaine (intra)cytoplasmique de l'enveloppe GALV.

Dans un mode préféré de mise en oeuvre, les protéines chimériques de l'invention comprennent donc tout ou partie du domaine extracellulaire de la protéine d'enveloppe GALV ou d'un variant fonctionnel de celui-ci. L'expression "tout ou partie" indique que les chimères de l'invention peuvent comprendre soit l'intégralité du domaine, soit une partie seulement de celui-ci. Au sens de l'invention, la partie du domaine extracellulaire peut comprendre avantageusement 50% au moins des résidus du domaine, de préférence 60% au moins, encore plus préférentiellement 75% au moins. Cette partie peut être obtenue par les techniques classiques de biologie moléculaire, impliquant par exemple la coupure enzymatique, la ligature, l'amplification, le clonage, etc, comme illustré dans la partie expérimentale.

Avantageusement, il s'agit d'une partie fonctionnelle du domaine considéré. A cet égard, le terme "variant fonctionnel" ou "partie fonctionnelle" du domaine extracellulaire, désigne plus spécifiquement toute partie ou variant conservant la capacité d'interagir avec le récepteur de l'enveloppe considérée. Le récepteur Glvr-1 (également désigné PiT-1) est le récepteur naturel de l'enveloppe GALV. Lorsque ce récepteur est présent à la surface des cellules, ces cellules sont permissives au virus GALV. Une propriété avantageuse des enveloppes chimériques selon l'invention est qu'elles présentent préférentiellement la capacité de liaison au récepteur Glvr-1.

La capacité de liaison au récepteur Glvr-1 des domaines extracellulaires (variants ou fragments de GALV) ou des enveloppes chimériques de l'invention peut être testée dans différentes conditions, et notamment par :
- incubation dudit domaine ou de l'enveloppe entière en présence du récepteur Glvr-1, et mise en évidence d'une fixation par les techniques classiques (marquage, compétition, etc.). Le récepteur utilisé peut être sous forme purifiée, le cas échéant fixé sur un support. Il peut également s'agir d'une cellule ou membrane cellulaire exprimant ledit récepteur ;
- par production de rétrovirus recombinants comprenant ladite enveloppe chimérique, et mise en évidence de la capacité desdits rétrovirus d'infecter une cellule porteuse du récepteur ; ou encore
- par toute autre technique connue de l'homme du métier.

En outre, le terme variant indique tout polypeptide comportant une ou plusieurs modifications de structure primaire, en particulier une ou plusieurs mutations, délétions, substitutions et/ou additions de résidus d'acides aminés, de préférence de moins de 30 acides aminés.

Plus préférentiellement, les protéines chimères de l'invention comprennent tout ou partie du domaine extracellulaire de la protéine d'enveloppe GALV représenté par les acides aminés 1 à 490 de la séquence SEQ ID NO:1 ou d'un variant fonctionnel de celui-ci.

Une variante particulière de l'invention consiste en une protéine chimère comprenant le domaine extracellulaire de la protéine d'enveloppe GALV représenté par les acides aminés 1 à 490 de la séquence SEQ ID NO:1.

Comme indiqué ci-avant, le domaine N-terminal des protéines chimères de l'invention peut en outre être composé de tout domaine protéique susceptible de conférer à l'enveloppe un tropisme particulier (autre enveloppe virale, enveloppe cellulaire, anticorps ou fragment ou dérivé d'anticorps, ligand, etc.).

Dans un premier mode particulier de mise en oeuvre, les protéines chimériques selon l'invention comprennent au moins une partie ou un variant fonctionnel du domaine extracellulaire de la protéine d'enveloppe GALV, un domaine transmembranaire hétérologue et un domaine cytoplasmique hétérologue. Une variante plus particulière réside dans une protéine d'enveloppe comprenant un domaine extracellulaire représenté par les acides aminés 1 à 490 de la séquence SEQ ID NO:1, plus un domaine transmembranaire et un domaine cytoplasmique hétérologues.

Dans un autre mode préféré de mise en oeuvre, les protéines chimériques selon l'invention comprennent au moins une partie du domaine extracellulaire et du domaine transmembranaire de l'enveloppe du virus GALV ou d'un variant fonctionnel de ceux-ci.

Avantageusement, les protéines chimères de l'invention comprennent tout ou partie des domaines extracellulaire et transmembranaire de la protéine d'enveloppe GALV représentés par les acides aminés 1 à 655 de la séquence SEQ ID NO:1 ou d'un variant fonctionnel de ceux-ci. Le terme variant fonctionnel est défini comme ci-avant. Dans le cas d'un domaine transmembranaire, il s'agit de tout variant conservant la capacité de s'insérer dans la membrane cellulaire.

Préférentiellement, les protéines chimères de l'invention comprennent l'intégralité du domaine extracellulaire de la protéine d'enveloppe GALV. Dans un autre mode préféré, les protéines chimères de l'invention comprennent l'intégralité du domaine transmembranaire de la protéine d'enveloppe GALV. Un mode de réalisation particulier de l'invention est représenté par une protéine d'enveloppe chimérique caractérisée en ce qu'elle comprend une région de l'enveloppe GALV consistant en la séquence correspondant aux résidus d'acides aminés 1 à 655 de la séquence SEQ ID NO:1.

Les protéines d'enveloppe selon la présente invention sont plus particulièrement caractérisées en ce qu'elle comprennent un domaine cytoplasmique d'une enveloppe hétérologue. Le terme "enveloppe hétérologue" désigne toute protéine d'enveloppe autre que la protéine d'enveloppe du virus GALV dont la séquence est représentée sur la SEQ ID NO:5. En particulier, il peut s'agir d'une enveloppe provenant d'un variant de GALV ayant une séquence différente de la séquence SEQ ID NO:5 (notamment un domaine cytoplasmique de séquence SEQ ID NO:4 ou un variant fonctionnel de celle-ci), d'une enveloppe d'un rétrovirus d'un autre type que GALV, d'une enveloppe cellulaire ou synthétique.

Par ailleurs, comme indiqué ci-avant, les protéines chimériques selon la présente invention peuvent également comporter un domaine transmembranaire hétérologue, c'est-à-dire tout domaine protéique ayant la capacité de s'insérer dans la membrane cellulaire, autre que le domaine transmembranaire de la protéine d'enveloppe du virus GALV dont la séquence est représentée sur la SEQ ID NO:1. Il peut s'agir du domaine transmembranaire d'une enveloppe hétérologue, ou également d'un domaine transmembranaire artificiel ou dérivé de toute protéine membranaire (récepteur, canal ionique, etc.). Préférentiellement, le domaine transmembranaire est un domaine de protéine d'enveloppe rétrovirale, notamment de GALV ou d'une enveloppe hétérologue.

La protéine d'enveloppe hétérologue peut être toute protéine d'enveloppe connue de l'homme de l'art, telle que par exemple la protéine d'enveloppe des virus 4070A, 10A1, VSV, VIH, du virus de la rage, du virus GALV, ou également une protéine d'enveloppe cellulaire.

Préférentiellement, la protéine d'enveloppe hétérologue est une protéine d'enveloppe d'un rétrovirus ayant un tropisme pour les cellules humaines, notamment un rétrovirus amphotrope. A cet égard, on peut citer la protéine d'enveloppe des rétrovirus 4070A, 10A1, etc. Une protéine d'enveloppe hétérologue particulièrement préférée au sens de l'invention est la protéine d'enveloppe du rétrovirus 4070A. Cette protéine d'enveloppe a été décrite dans la littérature et est couramment utilisée pour la construction de rétrovirus recombinants (voir notamment Ott et al., J. Virol. Vol. 64, p757-766, 1990). La séquence du domaine cytoplasmique de cette protéine est représentée sur la séquence SEQ ID NO: 6 : la partie cytoplasmique s'étend des acides aminés 635 à 667 (Figure 2) de la séquence SEQ ID NO: 6.

Dans un mode particulier de réalisation, l'invention concerne donc une protéine d'enveloppe chimérique, comprenant tout ou partie des domaines extracellulaire et transmembranaire de la protéine d'enveloppe GALV et le domaine cytoplasmique de l'enveloppe du rétrovirus 4070A, plus spécifiquement la séquence d'acides aminés du domaine cytoplasmique représenté de l'acide aminé 635 à l'acide aminé 667 dans la séquence SEQ ID NO: 6.

Dans un mode de réalisation spécifique, l'invention concerne une protéine d'enveloppe chimérique caractérisée en ce qu'elle comprend, en N-terminal, les résidus d'acides aminés 1 à 655 de la séquence SEQ ID NO:1 fusionnés aux résidus d'acides aminés 635 à 667 de la séquence SEQ ID NO: 6 (en C-terminal).

Dans un autre mode de réalisation, il s'agit d'une protéine d'enveloppe chimérique comprenant une région N-terminale déterminée, liée à la séquence comprise entre les résidus 491-685 de la séquence SEQ ID NO:2 ou un variant fonctionnel de celle-ci.

Une enveloppe chimérique non fusogène particulière de l'invention comprend, comme région C-terminale (iii), la séquence SEQ ID NO:4 ou un variant fonctionnel de celle-ci. Les variants fonctionnels de domaines cytoplasmiques selon l'invention, capables de conférer un caractère non-fusogène aux protéines, peuvent être construits par mutation(s), délétion(s), substitution(s) et/ou insertion(s), comme décrit ci-avant, puis testés sur le plan fonctionnel par un procédé comprenant :
- la liaison dudit domaine à un domaine extracellulaire et un domaine transmembranaire de protéines d'enveloppe (par exemple la région 1-655 de la séquence SEQ ID N0:2),
- mesure de l'activité fusogène de la protéine chimère obtenue sur des cellules possédant le récepteur correspondant au domaine extracellulaire utilisé. Le test de fusogénicité peut être réalisé comme décrit par Lavillette et al., 1998).

Selon l'invention, un variant est considéré comme fonctionnel si la fusogénicité de la protéine chimérique n'est par augmentée de plus de 25% par rapport à la même protéine comportant le domaine cytoplasmique de référence. A cet égard, de manière plus générale, cette méthodologie permet également, selon la présente invention, de déterminer la région cytoplasmique minimale des protéines d'enveloppe permettant d'éviter la fusogénicité. De telles régions minimales peuvent être utilisées comme région (iii) dans les protéines de l'invention, pour fabriquer des enveloppes non-fusogènes.

Les protéines d'enveloppe chimères selon l'invention comprennent donc avantageusement un domaine extracellulaire, un domaine transmembranaire et un domaine cytoplasmique. Ces différents domaines sont liés de manière fonctionnelle les uns aux autres, c'est-à-dire de sorte que chaque domaine conserve une activité dans la protéine chimérique. Généralement, les domaines sont directement liés les uns aux autres, sans l'intervention d'une molécule (peptide) espaceur. Néanmoins, il est possible que des régions neutres sur le plan fonctionnel soient intercalées entre les/des domaines, notamment du fait des techniques de synthèse utilisées. Ainsi, lorsque les acides nucléiques sont préparés, il est possible que des résidus nouveaux apparaissent, codés par exemple par des sites de restriction. Préférentiellement, de tels peptides espaceurs sont de taille très réduite, en particulier de 1 à 3 résidus.

L'invention concerne en outre toute composition comprenant une protéine ou un polypeptide de l'invention.

Un autre objet de l'invention réside également dans un acide nucléique codant pour une protéine telle que définie ci-avant. Il peut s'agir notamment d'un ADN (notamment ADNc) ou d'un ARN. Il peut en outre s'agir d'un acide nucléique de nature synthétique ou semi-synthétique, optimisé pour un usage de codons particulier, ou comprenant un ou plusieurs introns artificiels en vue de favoriser l'expression, etc. L'acide nucléique peut être préparé par les techniques classiques de la biologie moléculaire (criblage de banques, synthèse artificielle, amplification, coupures/ligations, clonages, etc.). Généralement, pour la constitution des acides nucléiques de l'invention, la région codant pour le ou les domaines de l'enveloppe GALV est préparée, puis assemblée à la région codant pour le(s) domaine(s) de l'enveloppe hétérologue.

Ainsi, un objet particulier de l'invention réside également dans un acide nucléique codant pour une protéine d'enveloppe GALV comprenant une extrémité C-terminale de séquence SEQ ID NO:3 telle que définie ci-avant, plus particulièrement un domaine cytoplasmique de séquence SEQ ID NO:4 tel que défini ci-avant. Un mode spécifique de réalisation comprend un acide nucléique tel que représenté sur la séquence SEQ ID NO:1, du nucléotide en position 1 jusqu'à un nucléotide compris entre les positions 2056 et 2129 inclus de la séquence SEQ ID NO: 1.

Des modes de réalisation plus spécifiques de l'invention sont représentés par:
- un acide nucléique codant pour une protéine d'enveloppe GALV, caractérisé en ce qu'il comprend une séquence s'étendant du nucléotide en position 1 jusqu'à un nucléotide compris entre les positions 2056 et 2112 inclus de la séquence SEQ ID NO: 1;
- un acide nucléique codant pour une protéine d'enveloppe GALV, caractérisé en ce qu'il comprend une séquence s'étendant du nucléotide en position 1 jusqu'au nucléotide 2058 de la séquence SEQ ID NO: 1 ;

L'invention concerne également les variants des séquences ci-dessus, tels que notamment les séquences résultant de la dégénérescence du code génétique et codant pour une protéine de même structure primaire, les variants résultant du polymorphisme du virus GALV, les variants présentant des altérations génétiques et codant pour une protéine d'enveloppe de type GALV fonctionnelle, c'est-à-dire essentiellement non-fusogénique et capable de lier le récepteur Glvr-1. L'invention concerne en outre toute séquence hybridant avec les séquences ci-dessus, de préférence dans des conditions de stringence élevée, et codant pour une protéine d'enveloppe de type GALV fonctionnelle.

La présente invention est également relative à tout vecteur comprenant un acide nucléique tel que défini ci-dessus. Un vecteur selon l'invention peut être de nature plasmidique, épisomique, chromosomique, virale, etc. Il s'agit plus particulièrement d'un vecteur plasmidique, par exemple construit à partir de plasmides connus tels que pBR, pUC, pBS, pCl, etc. Avantageusement, dans les vecteurs, les acides nucléiques de l'invention sont placés sous le contrôle d'un promoteur transcriptionnel. Il peut s'agir à cet effet d'un promoteur transcriptionnel fort ou faible, constitutif ou sélectif, régulé ou non, d'origine cellulaire (procaryote, eucaryote, y compris mammifère, dont humain), virale ou artificielle. Des promoteurs utilisables à cet effet sont par exemple les promoteurs CMV, PGK, TK, SV40, etc. Le choix du promoteur peut être aisément réalisé par l'homme de l'art en fonction des applications recherchées.

Préférentiellement, le vecteur de l'invention comprend en outre une origine de réplication, notamment une origine de réplication fonctionnelle dans les cellules procaryotes, permettant une manipulation et une production aisées des vecteurs dans ce type d'hôtes (par exemple dans les bactéries, notamment E. coli). Par ailleurs, le vecteur peut également comprendre un gène marqueur, permettant de sélectionner les cellules dans lesquelles le vecteur a été introduit. Il peut s'agir notamment d'un gène de résistance à un composé (par exemple à un antibiotique).

Un autre objet de l'invention concerne toute cellule comprenant un acide nucléique ou un vecteur tels que définis ci-avant. Une telle cellule peut être procaryote ou eucaryote. En outre parmi les eucaryotes, il peut s'agir d'un eucaryote inférieur (par exemple une levure) ou d'un eucaryote supérieur (cellule végétale, animale, humaine, etc.).

Avantageusement, l'invention concerne toute cellule de packaging de rétrovirus, caractérisée en ce qu'elle comprend un acide nucléique ou un vecteur tels que définis ci-avant et un acide nucléique codant pour les protéines rétrovirales gag et pol. Une telle cellule de packaging est préférentiellement une cellule de mammifère, telle qu'un fibroblaste, d'insecte, ou bien, de préférence, humaine (cellule embryonaire par exemple). Pour cet objet plus particulier, on utilise avantageusement des cellules cultivables, non-pathogènes, et transfectables par des acides nucléiques. Des exemples particuliers sont des cellules embryonaires de rein ou de rétine humaine, ou de lignées cancéreuses humaines.

Pour la préparation des cellules de l'invention, les acides nucléiques ou vecteurs peuvent être introduits dans les cellules par toute technique connue de l'homme du métier (ADN "nu", électroporation, précipitation au phosphate de calcium, "gene gun", vecteur lipidique, cationique, polymérique, etc.). Après incubation, les cellules sont sélectionnées, soit sur la base de l'expression de l'enveloppe, soit sur la base de l'expression du gène marqueur (par exemple par culture en milieu antibiotique). Ces cellules peuvent alors être maintenues en culture, ou clonées et/ou conservées sous forme congelée, par exemple.

L'invention réside également dans l'utilisation d'un acide nucléique ou d'un vecteur tels que définis ci-avant pour la production d'une cellule de packaging de rétrovirus. L'invention réside en outre dans une méthode de production d'une cellule de packaging de rétrovirus, comprenant l'introduction, dans une cellule compétente, d'un acide nucléique ou d'un vecteur tels que définis ci-avant et d'un ou plusieurs acides nucléiques codant pour les protéines rétrovirales gag et pol. Les différents acides nucléiques peuvent être introduits de manière simultanée ou séquentielle, dans un ordre indifférent. Les cellules compétentes sont toutes cellules telles que définies ci-avant.

Les protéines d'enveloppe de type-GALV de l'invention telles que décrites ci-avant présentent des propriétés fonctionnelles avantageuses, telles que notamment une absence de fusogénicité. Ainsi, alors que les protéines GALV connues jusqu'à présent induisent une fusion de cellules humaines les produisant, les protéines d'enveloppe chimériques de l'invention sont préférentiellement dépourvues de cet inconvénient. Ces enveloppes peuvent donc être utilisées avantageusement pour la production de rétrovirus recombinants dans des cellules humaines. Ceci représente un avantage important par rapport à l'art antérieur, puisque les rétrovirus produits dans les lignées classiques (fibroblastes murins) possèdent des déterminants antigéniques murins et sont donc potentiellement immunogènes.

A cet égard, l'invention a aussi pour objet un procédé de production de rétrovirus recombinants défectifs, comprenant l'introduction, dans une cellule de packaging selon l'invention, d'un vecteur rétroviral, et la récupération des virus recombinants produits. Les virus produits peuvent être récupérés par toute technique classique, telle que récolte du surnageant, éventuellement purification des rétrovirus par des étapes de centrifugation et/ou chromatographie. L'invention a également pour objet tout rétrovirus comprenant, dans sa structure, une protéine d'enveloppe telle que décrite ci-avant. Il s'agit plus particulièrement d'un rétrovirus recombinant, en particulier défectif, par exemple produit selon le procédé décrit ci-dessus.

Par ailleurs, comme indiqué ci-avant, un autre aspect de la présente invention réside dans l'utilisation de protéines d'enveloppes, notamment rétrovirales, fusogéniques pour induire la destruction de cellules.

Ainsi, l'invention décrit également la construction d'enveloppes rétrovirales modifiées, fusogènes, et leur utilisation comme molécules toxiques pour induire la destruction de cellules in vitro, ex vivo ou in vivo.

En effet, la présente invention montre qu'il est possible de modifier la structure des protéines d'enveloppe, notamment rétrovirales pour produire des polypeptides fusogènes. La présente invention réside également dans l'utilisation des ces propriétés fusogéniques comme agents de toxicité cellulaire. Cette utilisation est avantageuse puisque, contrairement à certaines toxines connues et utilisées, elle ne se manifeste pas dans certains types cellulaires et peut donc (i) permettre une production en quantités importantes dans des cellules résistantes et (ii) permettre une destruction efficace de cellules in vitro, ex vivo ou in vivo.

L'invention a donc également pour objet l'utilisation de protéines d'enveloppe fusogène (ou de tout acide nucléique codant de telles protéines) comme agent de toxicité cellulaire, i.e., pour induire une destruction de cellules, in vitro, ex vivo ou in vivo. L'invention concerne notamment l'utilisation de protéines d'enveloppe fusogène pour la préparation de compositions (pharmaceutiques) destinées à la mise en oeuvre d'une méthode de traitement thérapeutique et/ou vaccinal, notamment pour le traitement de cellules humaines in vitro, ex vivo ou in vivo. L'invention concerne également une méthode de destruction de cellules comprenant la mise en contact de cellules avec une enveloppe fusogène ou tout acide nucléique codant une telle enveloppe. L'invention concerne également une méthode de traitement de cellules pour rendre cellules-ci fusogènes, comprenant la mise en contact de cellules avec une enveloppe fusogène ou tout acide nucléique codant une telle enveloppe.

Cette application de l'invention peut être mise en oeuvre aussi bien pour induire la destruction de cellules, que pour faciliter leur fusion. Ainsi, cette application peut être utilisée pour réaliser (ou faciliter) la fusion de cellules immortalisées (par exemple tumorales) avec des cellules d'intérêt (des cellules productrices d'anticorps ou présentatrices d'antigènes, par exemple). Un exemple particulier concerne la préparation d'hybridomes par fusion de cellules productrices d'anticorps avec des cellules immortalisées (par exemple de myelome). En présence de protéines fusogènes de l'invention, cette fusion est favorisée et le procédé de préparation d'hybridomes est amélioré. Il peut bien entendu s'agir d'un procédé de préparation de toute fusion cellulaires, entre des cellules animales, insectes ou végétales, possédant le récepteur du domaine extracellulaire de l'enveloppe utilisée.

Plus spécifiquement, les protéines d'enveloppe fusogènes selon l'invention sont des protéines de type rétroviral, c'est-à-dire dérivées de protéines d'enveloppe rétrovirales. Généralement, il s'agit de protéines d'enveloppe rétrovirales modifiées dans la région C-terminale. En effet, la présente invention montre à présent que la modification de l'extrémité C-terminale d'une protéine d'enveloppe rétrovirale peut induire l'apparition de propriétés fusogènes chez cette protéine. Ainsi, la présente demande montre notamment que le domaine cytoplasmique des protéines d'enveloppe rétrovirales comporte des régions d'homologie significative, et que la modification (en particulier la suppression) de tout ou partie de ce domaine conduit à l'apparition de propriétés fusogènes.

Ainsi, la présente invention réside dans l'utilisation de protéines d'enveloppe rétrovirales modifiées, fusogènes, pour traiter des cellules et/ou induire leur destruction. Plus particulièrement, il s'agit de protéines d'enveloppe rétrovirales fusogènes modifiées dans la région C-terminale, notamment dans le domaine cytoplasmique. Encore plus particulièrement, il s'agit de protéines d'enveloppe rétrovirales fusogènes comprenant une délétion de tout ou partie de leur domaine (intra)cytoplasmique.

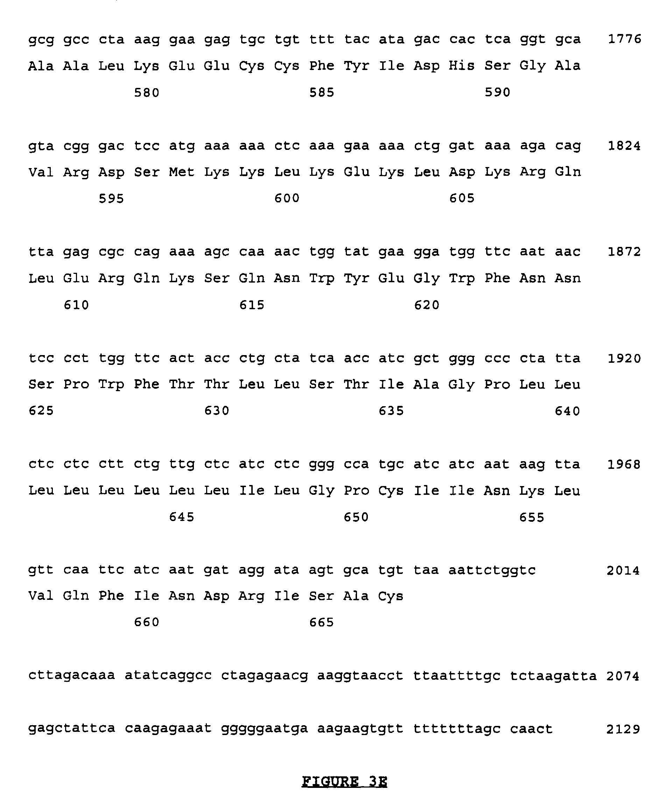

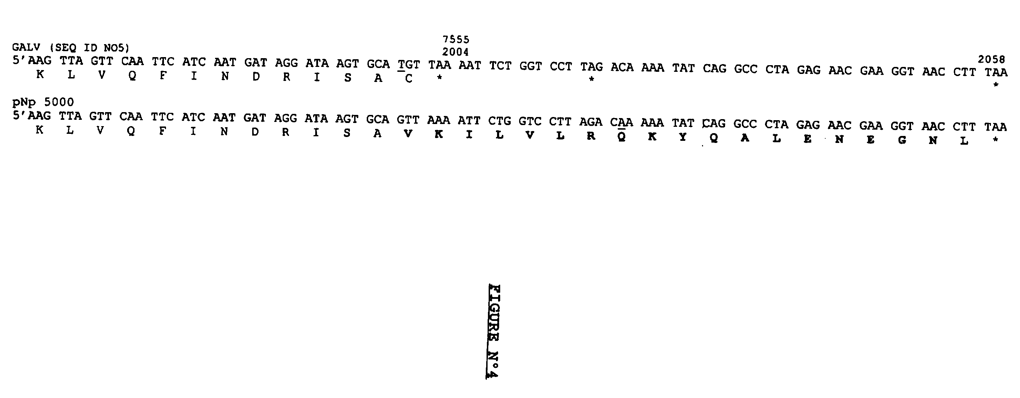

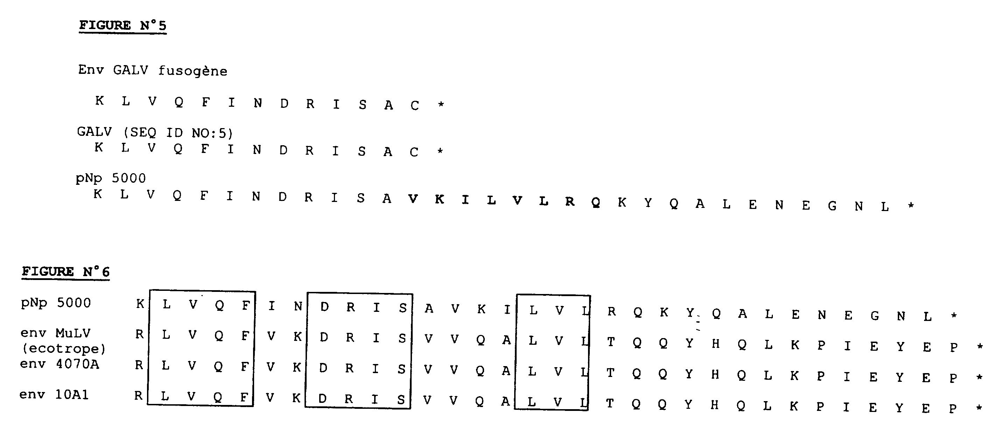

De telles protéines d'enveloppe peuvent être préparées à partir d'enveloppe différentes, notamment rétrovirales, telles que mentionnées ci-avant. Il peut s'agir notamment d'une enveloppe d'un rétrovirus ayant un tropisme pour les cellules humaines, ce qui permet de générer une protéine fusogénique susceptible d'induire la destruction de cellules humaines. A cet égard, on peut citer plus spécifiquement les enveloppes de rétrovirus de type C, tels que GALV, 4070A, 10A1, MuLV, VSV, VIH ou du virus de la rage. Comme illustré sur la figure 6, les domaines cytoplasmiques des protéines d'enveloppe GALV, 4070A, 10A1, MuLV présentent des régions d'homologie forte, qui peuvent être modifiées pour produire des molécules fusogènes.

L'invention concerne aussi un procédé de préparation d'une protéine fusogène, comprenant (i) la modification de la région cytoplasmique d'une protéine d'enveloppe, notamment rétrovirale, et (ii) la mise en évidence de l'activité fusogène de la protéine, par exemple par incubation en présence de cellules exprimant le récepteur de l'enveloppe. La modification de la région cytoplasmique de la protéine comprend plus spécifiquement la délétion de tout ou partie de cette région, notamment d'un fragment de l'extrémité C-terminale comprenant au moins 5 acides aminés, de préférence au moins 10 acides aminés, encore plus préférentiellement au moins 15 acides aminés. La modification peut également comprendre toute autre inactivation, telle que des mutations et/ou insertions inactivantes dans la région concernée.

Dans un mode particulier de mise en oeuvre, la protéine d'enveloppe est une protéine d'enveloppe de rétrovirus de type C, notamment GALV, 4070A, 10A1 ou MuLV et la modification comprend la suppression d'une région du domaine cytoplasmique comprenant au moins tout ou partie des résidus situés du coté C-terminal du motif LVL.

Dans un autre mode de réalisation, la protéine d'enveloppe fusogène de l'invention est une protéine chimérique comprenant un domaine extracellulaire ayant un tropisme désiré, un domaine transmembranaire, et un domaine cytoplasmique construit comme décrit ci-dessus, de manière à conférer à ladite protéine une activité fusogène. Ces différents domaines peuvent êtreconstruits et assemblés comme décrits ci-avant pour les chimères non-fusogènes.

Les protéines d'enveloppe fusogènes ainsi construites et testées peuvent être produites, de préférence sous forme recombinante. Préférentiellement, elles sont produites à partir d'un vecteur rétroviral (sous forme de particules rétrovirales incorporant un tel vecteur). Dans ce mode de réalisation, un vecteur rétroviral comprenant une séquence nucléique codant une protéine fusogène selon l'invention est introduit dans une lignée de packaging de rétrovirus, résistante à la fusogénicité. Une telle lignée est généralement une lignée de cellules n'exprimant pas (ou peu) le récepteur de l'enveloppe. Ainsi, dans le cas particulier de l'enveloppe GALV, on peut utiliser une lignée de packaging murine. Par ailleurs, il peut s'agir d'une lignée de packaging modifiée pour ne plus exprimer de récepteur fonctionnel à la protéine d'enveloppe considérée. Ainsi, dans le cas particulier de l'enveloppe GALV, on peut envisager d'utiliser une lignée de packaging d'origine humaine dont le génome a été modifié par inactivation du gène codant le récepteur de l'enveloppe (i.e., le récepteur Glvr-1 de GALV). La lignée de packaging utilisée est une lignée exprimant les régions gag et pol de rétrovirus, et éventuellement la région env d'un rétrovirus. Le vecteur rétroviral décrit ci-dessus, comprenant une séquence nucléique codant une protéine fusogène selon l'invention constitue un autre objet de la présente invention. Un tel vecteur comprend avantageusement une séquence LTR à chaque extrémité et un site d'encapsidation.

Lorsque la protéine d'enveloppe est exprimée à partir d'une particule rétrovirale, la mise en contact des cellules à traiter (notamment à détruire) avec l'enveloppe fusogène est réalisée par mise en contact des cellules in vitro, ex vivo ou in vivo avec les particules rétrovirales. L'infection des cellules conduit à l'expression de l'enveloppe dans les membranes cellulaires. Ces protéines d'enveloppe sont ensuite capables d'induire la fusion des cellules traitées avec tout cellule exprimant le récepteur de l'enveloppe.

A titre d'exemple plus spécifique, le vecteur rétroviral ainsi produit peut être introduit dans des cellules humaines cibles (par exemple des cellules cancéreuses, des cellules infectées par un virus ou produites dans le cas de maladies autoimmunes) pour générer une réaction toxique pour ladite cellule, résultant du caractère fusogénique de l'enveloppe.

Bien entendu, les protéines d'enveloppe fusogènes peuvent également être produites par expression à partir de tout autre système vecteur, notamment à partir d'un vecteur plasmique ou viral. Un exemple particulier est un vecteur adénoviral comprenant un acide nucléique codant une enveloppe telle que définie ci-avant. La préparation de tels adénovirus peut être réalisée selon les techniques connues de l'homme du métier, par exemple par délétion de tout ou partie de la région E1, E2, E3 et/ou E4 du génome adénoviral, insertion dans ce génome de l'acide nucléique codant, puis introduction du génome ainsi préparé dans une cellule de packaging appropriée, par exemple dérivée des cellules 293, le cas échéant en présence de plasmide(s) ou virus helper. Dans ce mode de mise en oeuvre, il n'est pas nécessaire que la lignée de packaging utilisée soit dépourvue du récepteur d'enveloppe, puisque la phase de production conduit à une lyse des cellules par les adénovirus.

Plus particulièrement, cette utilisation selon l'invention peut être appliquée à la destruction de cellules sensibles à l'enveloppe utilisée, (par exemple GALV), en particulier de cellules possédant le récepteur de l'enveloppe utilisée (le récepteur Glvr-1 pour GALV). Les propriétés fusogènes peuvent ainsi être appliquées au traitement de cellules humaines pathologiques (cancéreuse, resténose, autoimmunes, etc.).

D'autres aspects et avantages de la présente invention apparaîtront à la lecture des exemples qui suivent, qui doivent être considérés comme illustratifs et non limitatifs.

### Légende des Figures

### FIGURE 1 :

Séquence de l'enveloppe GALV (correspondant à la partie 3' du génome du virus), de l'acide aminé en position 1 à l'acide aminé en position 685 (SEQ ID NO: 1).

### FIGURE 2 :

Séquence de la protéine d'enveloppe du rétrovirus 4070A (SEQ ID NO:6). Le domaine cytoplasmique s'étend des résidus 635 à 667.

### FIGURE 3 :

Séquence de la partie 3' du génome du virus GALV telle que décrite dans l'art antérieur (SEQ ID NO:5). La région correspondant à la protéine d'enveloppe s'étend du nucléotide 1 au nucléotide 2004, correspondant aux nucléotides 5552 à 7555 du génome.

### FIGURE 4 :

Comparaison de la séquence nucléique et protéique du domaine cytoplasmique des protéines d'enveloppe de séquence SEQ ID NO:1 et 5.

### FIGURE 5 :

Comparaison de la séquence protéique du domaine cytoplasmique des protéines d'enveloppe de séquence SEQ ID NO:2 et 5.

### Figure 6 :

Comparaison de la séquence d'acides aminés de domaines cytoplasmiques de protéines d'enveloppe de rétrovirus de type C.

Dans la description, les exemples et les revendications, il est fait référence au Listing de Séquences, dont le texte libre est reproduit ci-dessous :
<223> Enveloppe GALV
<223> Enveloppe GALV
<223> Extrémité C-terminale Enveloppe GALV
<223> Domaine Cytoplasmique Enveloppe GALV
<223> Enveloppe GALV
<223> Enveloppe amphotrope 4070A
<223> Oligonucléotide OUGalv5' Nsi
<223> Oligonucléotide OLCT1Cla
<223> Oligonucléotide OLCT2Cla
<223> Oligonucléotide OLCT3Cla
<223> Oligonucléotide OLCT4Cla
<223> Oligonucléotide 5' Nhe1
<223> Oligonucléotide 3' Xba1
<223> Oligonucléotide 3' Xba1

### Exemples

### Exemple 1

Constructions génétiques codant pour une enveloppe GALV non fusogénique.

### 1.1. Plasmides et constructions.

Le plasmide CMV-GALV (Chapel-Fernandes et al., 1998) exprime le gène env codant pour la glycoprotéine d'enveloppe du rétrovirus GALV (gibbon ape leukemia virus). Les sites Nsil et Clal sont uniques à l'extrémité 3' du gène env et ont été utilisés pour cloner les fragments suivants :
- CT1, dont l'extrémité 3' (nt 7663) est dans le LTR (long terminal repeat, début: nt 7651) de GALV,
- CT2, CT3, et CT4 dont les extrémités 3' (respectivement aux positions 7606, 7588 et 7552) sont dans la région située entre le gène env et le LTR. Les positions ci-dessus sont données par référence à la séquence génomique de GALV.

Ces fragments sont obtenus par PCR au moyen d'un oligonucleotide "upper" commun (OUGalv5'Nsi), et d'oligonucléotides "lower" uniques pour chaque fragment (OLCT1Cla, OLCT2Cla, OLCT3Cla, et CLCT4Cla), apportant un codon stop dans les trois cadres de lecture et en particulier celui propre au gène env, suivis d'un site Clal. Les séquences des oligonucléotides, obtenus à partir de la séquence du GALV, sont :
OUGalv5'Nsi (SEQ ID NO: 7) : 5'-GAGCGCCAGAAAAGCCAAAACTGGTATGAAGG OLCT1Cla (SEQ ID NO: 8) : 5'- GATCATCGATTATTTATTTACACTTCTTTCATTCCCCCATTTCTCTTGTG OLCT2Cla (SEQ ID NO: 9) : 5'-GATCATCGATTATTTATTTAAAGGTTACCTTCGTTCTCTAGGGCCTGA OLCT3CIa (SEQ ID NO: 10): 
5'-GATCATCGATTATTTATTTATAGGGCCTGATATTTTGTCTAAGGACCA
GAA
 OLCT4Cla (SEQ ID NO: 11):

5'- GATCATCGATTATTTATTTAACATGCACTTATCCTATCATTGATGAAT
TGA

Les fragments d'ADN obtenus par PCR ont été ensuite purifiés, digérés par les enzymes Nsil et Clal, et clonés dans le plasmide CMV-GALV ouvert aux sites Nsil et Clal. Les quatre plasmides résultants codent pour les enveloppes dénommées GALV-CT1, GALV-CT2, GALV-CT3 et CMV-GALV-CT4. Les plasmides FBASALF (Cosset et al., J. Virol. 69 (1995) 7430) et FBARIessSALF (Lavillette et al., J. Virol. 1998, In press) codent pour des enveloppes amphotropes, non fusogéniques (enveloppe A) et fusogéniques (enveloppe Arless), respectivement. Ces plasmides sont utilisés comme contrôles négatifs et positifs dans les tests de syncytium.

### 1.2 Cellules et tests de fusion.

Les cellules humaines TE671 sont issues d'un rhabdomyosarcome humain (ATCC CRL8805), et les cellules NIH-3T3 sont des fibroblastes de souris.

Les tests de fusions sont réalisés comme publié antérieurement (Lavillette et al., 1998). Les cellules TE671 ensemencées à 5x10⁶ cellules dans des boîtes de 35 mm de diamètre sont transfectées avec 2pg des divers plasmides par la technique de phosphate de calcium. 48 hrs post-transfection, les cellules sont trypsinées et ré-ensemencées dans des boîtes de 35 mm de diamètre à une densité de 2x10⁶ cellules par boîte. Après adhésion de ces cellules productrices de glycoprotéines d'enveloppes, des cellules indicatrices (soit TE671, soit 3T3) sont ajoutées, à une densité de 2x10⁶ cellules par boîte. Les cocultures sont alors maintenues pendant 24-48 hrs et les syncytium sont comptés après fixation au May-Grunwald-Giemsa.

Les résultats obtenus sont présentés dans le tableau 1 ci-dessous :

**Tableau 1**

| Enveloppes^{a} | Fusogénicité (Syncytia)^{b} | |
|---|---|---|
| | 3T3 | TE671 |
| - | - | - |
| A | - | - |
| Arless | + | + |
| GALV-CT1 | - | - |
| GALV-CT2 | - | - |
| GALV-CT3 | - | +/- |
| GALV-CT4 | - | + |

| | | |
|---|---|---|
| a : Enveloppe exprimée dans les TE671 transfectées. | | |
| b : Formation de syncytium dans les cocultures des cellules productrices d'enveloppes avec les 3T3 ou avec les TE671 | | |

### 1.3. Conclusions

Les résultats présentés dans le Tableau 1 ci-dessus montrent que l'enveloppe GALV de type sauvage, exprimée à partir d'une construction génétique constituée essentiellement de la séquence codante (i.e., jusqu'au codon stop en position 2004) présente une phénotype fusogénique important dans les cellules humaines (GALV-CT4). En revanche, de manière surprenante, l'enveloppe GALV codée par un vecteur d'expression dans lequel la séquence issue de GALV contient le gène env et une partie au moins de la région située entre ce gène et le LTR3' (ainsi que, le cas échéant, un morceau de LTR3') n'est pas fusogénique en test de syncytium (GALV-CT1, GALV-CT2). Quand on étend cette séquence à partir de l'extrémité 3' (enveloppe CT4 à CT1), on révèle donc progressivement un phénotype non-fusogénique. On en déduit qu'il existe, en 3' de la séquence publiée du gène env, une séquence qui contrôle négativement la fusogénicité de l'enveloppe. Cette fusogénicité semble être spécifique du résultat d'une interaction avec le récepteur du GALV (PiT-1) car seules les cellules PiT-1 positives (les TE671) sont capables de former des syncytia.

### Exemple 2 :

Constructions génétiques codant pour une enveloppe GALV non-fusogénique.

### 2.1. Constructions génétiques

### . Plasmide pGALV-1

L'ADNc de l'enveloppe de GALV a été cloné par PCR à partir du vecteur d'expression pMOV GALV (Miller et al., J. Virol. 65 (1991) 2220) dans le plasmide commercial pCl (Stratagène) aux sites Nhe1 et Xbal. L'ADNc cloné à une taille de 2004 paires de bases (nt 5552 (ATG du gène) au nt 7555 (codon stop)).
Oligonucléotide 5' Nhe1 (SEQ ID NO: 12) 5'-GCTAGCATGGTATTGCTGCCTGGGTCCATGCTTCTCACCT-3' Oligonucléotide 3' Xba1 (SEQ ID NO: 13)

5'-TCTAGATTAACATGCACTTATCCTATCATTG

### . Plasmide pGALV-2

L'ADNc du gène de l'enveloppe GALV est obtenu par PCR, la partie 3' du gène est prolongée jusqu'au site Fst1 qui se situe dans le long terminal repeat (LTR). L'ADNc cloné dans le plasmide pC1 aux sites Nhe1-Xba1 a une taille de 2118 paires de bases (nt 5552 au nt 7670).
Oligonucléotide 5' Nhe1 (SEQ ID NO: 12) 5'-GCTAGCATGGTATTGCTGCCTGGGTCCATGCTTCTCACCT-3' Oligonucleotide 3' Xba1 (SEQ ID NO: 14)

5'-TCTAGAAGTTGGCTAAAAAAAAACACTTCTTTCATTCC-3'

### . Plasmide témoin

Le plasmide pCMV-4070A est le plasmide pCl dans lequel est cloné dans les sites Nhe1-Xba1, l'ADNc du gène de l'enveloppe amphotrope 4070A (nt 37 au nt 2001, Ott et al. 1990).

### 2.2. Lignées cellulaires

La lignée cellulaire humaine H293 est une lignée cellulaire embryonaire de rein (ATCC CRL-1573). Les cellules NIH-3T3 sont des fibroblastes de souris.

Les cellules TelCeb6 sont des cellules dérivées de la lignée humaine TE671 issues d'un rhabdomyosarcome humain (ATCC CRL 8805) (Cosset FL, Lyon).

### 2.3. Etude de fusogénicité

Les plasmides p-GALV-1 et p-GALV-2 ont été transfectés dans les cellules H293, NIH-3T3 et TelCeb6 par la méthode de phosphate de calcium.

5pg de plasmides ont été transfectés dans chaque lignée cellulaire en boite de petri de 60 mm. 24 h après la transfection le milieu est changé. La présence ou l'apparition de fusion cellulaire sont observées dans les 48 h à 72 h post transfection, selon la méthode décrite dans l'exemple 1.2. Les résultats obtenus sont présentés dans le Tableau 2 ci-dessous.

**Tableau 2**

| Plasmides | fusogénicité sur NIH-3T3 | Fusogénicité sur Telceb6 | Fusogénicité sur H293 |
|---|---|---|---|
| pas de plasmide | 0 | 0 | 0 |
| pCMV-4070a | 0 | 0 | 0 |
| pGALV-1 | 0 | ++ | ++ |
| pGALV-2 | 0 | 0 | 0 |

### 2.4. Conclusion

Nous avons montré que l'enveloppe GALV (exprimée à partir du plasmide pGALV-1) pouvait entraîner une forte fusogénicité des cellules qui possèdent le récepteur PiT-1 du GALV (cellules Telceb6 et H293). Les résultats obtenus montrent que cette fusogénicité est abolie quand on prolonge la partie 3' de la séquence exprimée, notamment de 100 pb environ (pGALV2).

### Exemple 3

Pour caractériser plus précisément les mécanismes de fusogénicité, un gène de l'enveloppe GALV a été cloné (plasmide pNp5000) et sa séquence a été réalisée et analysée. De manière surprenante, cette analyse a permis de mettre en évidence un domaine C-terminal étendu par rapport à la séquence décrite dans l'art antérieur. La séquence protéique de cette enveloppe, présentée sur la séquence SEQ ID NO:2, comprend en effet 685 résidus, et fait apparaître une extrémité C-terminale comprenant la séquence Val-Lys-Ile-Leu-Val-Leu-Arg-Gln-Lys-Tyr-Gln-Ala-Leu-Glu-Asn-Glu-Gly-Asn-Leu (SEQ ID NO:3). Plus particulièrement, le domaine intracytoplasmique de cette protéine d'enveloppe comprend la séquence Lys-Leu-Val-Gln-Phe-lle-Asn-Asp-Arg-Ile-Ser-Ala-Val-Lys-Ile-Leu-Val-Leu-Arg-Gln-Lys-Tyr-Gln-Ala-Leu-Glu-Asn-Glu-Gly-Asn-Leu (SEQ ID NO:4). Une comparaison de la séquence du domaine intracytoplasmique de la protéine d'enveloppe déterminée ci-dessus avec la séquence SEQ ID NO:5 est représentée sur les figures 4 et 5.

Il est intéressant de noter que le domaine C-terminal identifié ci-dessus est absent des constructions GALV-CT4 et pGALV1, qui expriment une enveloppe GALV fusogénique (cf. Tableaux 1 et 2), alors que la séquence SEQ ID NO:1 (nt 1 à 2058) exprime une enveloppe non fusogénique. Ces résultats indiquent que l'intégrité de ce domaine est importante dans la manifestation des propriétés fusogéniques de l'enveloppe GALV. Ces résultats montrent aussi que la modification de la région C-terminale, notamment du domaine cytoplasmique, de la protéine GALV, permet de construire des protéines fusogéniques, utilisables comme agents toxiques.

Dans cette perspective, une comparaison de la séquence des domaines C-terminaux de différentes protéines d'enveloppe a été entreprise. Ainsi, la figure 6 montre l'alignement des séquences protéiques des domaines cytoplasmiques de plusieurs enveloppes de rétrovirus de type C. Cet alignement fait apparaitre certaines homologies, comme notamment la présence d'un site consensus de clivage LVL. La présence de ces homologies sugggère que l'altération des domaines cytoplasmiques des protéines d'enveloppe, notamment rétrovirales, permet de produire des protéines fusogéniques, comme dans le cas de l'enveloppe GALV.

### Exemple 4

Constructions génétiques codant pour une enveloppe de type GALV chimérique.

### 3.1. Construction du plasmide pGALV-3

La partie cytoplasmique du gène de l'enveloppe GALV (à partir du nt 7501 de la séquence SEQ ID NO 1 ou 5) a été remplacée par la partie cytoplasmique du gène de l'enveloppe du virus amphotrope 4070A (nt 1903 au nt 2001), SEQ ID NO: 6. L'ADNc cloné dans le plasmide pCl a donc une taille de 2061 paires de bases.

Cet ADN chimérique a été construit comme suit :
1) par PCR on a obtenu un fragment s'entendant de l'acide aminé 635 à l'acide aminé 667 de la séquence SEQ ID NO: 6.
2) on a remplacé les acides aminés 655 à 668 de la séquence SEQ ID NO: 5 ou 655 à 685 de la séquence SEQ ID NO: 2 par le fragment obtenu dans l'étape 1) ci-dessus.

### 3.2. Etude de fusogénicité

Les plasmides p-GALV-1 et p-GALV-3 ont été transfectés dans les cellules H293, NIH-3T3 et TelCeb6 par la méthode de phosphate de calcium. 5µg de plasmides ont été transfectés dans chaque lignée cellulaire en boite de petri de 60 mm. 24 h après la transfection le milieu est changé. La présence ou l'apparition de fusion cellulaire sont observées dans les 48 h à 72 h post transfection, selon la méthode décrite dans l'exemple 1.2. Les résultats obtenus sont présentés dans le Tableau 3 ci-dessous.

**Tableau 3**

| Plasmides | fusogénicité sur NIH-3T3 | Fusogénicité sur Telceb6 | Fusogénicité sur H293 |
|---|---|---|---|
| pas de plasmide | 0 | 0 | 0 |
| pCMV-4070a | 0 | 0 | 0 |
| pGALV-1 | 0 | ++ | ++ |
| pGALV-3 | 0 | 0 | 0 |

Ces résultats montrent de manière surprenante qu'une enveloppe chimérique, comprenant un domaine extracellulaire et transmembranaire fonctionnel de type GALV, et un domaine cytoplasmique hétérologue, est dépourvue de phénotype fusogénique, lorsqu'elle est exprimée dans une cellule humaine exprimant le récepteur PiT-1.

Ces résultats illustrent les propriétés avantageuses des méthodes et compositions selon la présente invention.

### Annexe à la description

## Revendications

1. Protéine d'enveloppe chimérique caractérisée en ce qu'elle comprend au moins une partie du domaine extracellulaire de l'enveloppe du virus GALV ou d'un variant fonctionnel de celui-ci.

2. Protéine d'enveloppe chimérique selon la revendication 1, caractérisée en ce qu'elle comprend au moins une partie du domaine extracellulaire et du domaine transmembranaire de l'enveloppe du virus GALV.

3. Protéine d'enveloppe chimérique selon la revendication 1 ou 2, caractérisée en ce qu'elle comprend un domaine cytoplasmique d'une enveloppe hétérologue.

4. Protéine d'enveloppe chimérique selon la revendication 3, caractérisée en ce que l'enveloppe hétérologue est une enveloppe amphotrope.

5. Protéine d'enveloppe chimérique selon la revendication 4, caractérisée en ce que l'enveloppe amphotrope est l'enveloppe 4070A.

6. Protéine d'enveloppe chimérique, caractérisée en ce qu'elle comprend une région de l'enveloppe GALV consistant en tout ou partie de la séquence correspondant aux résidus 1 à 655 de la séquence SEQ ID NO: 1.

7. Protéine d'enveloppe chimérique caractérisée en ce qu'elle comprend une région consistant en la séquence correspondant aux résidus 1 à 655 de la séquence SEQ ID NO: 1 et une région consistant en la séquence correspondant aux résidus 635 à 667 de la séquence SEQ ID NO: 6.

8. Protéine d'enveloppe rétrovirale GALV, caractérisée en ce qu'elle comprend une extrémité C-terminale de séquence SEQ ID NO:3.

9. Protéine d'enveloppe rétrovirale GALV, caractérisée en ce qu'elle comprend un domaine intracytoplasmique de séquence SEQ ID NO:4.

10. Protéine d'enveloppe rétrovirale GALV, caractérisée en ce qu'elle comprend la séquence d'acides aminés entre les résidus 1 à 685 de la séquence SEQ ID NO:1.

11. Acide nucléique codant pour une protéine d'enveloppe selon l'une des revendications 1 à 10.

12. Acide nucléique codant pour une protéine d'enveloppe GALV, caractérisé en ce qu'il comprend une séquence s'étendant du nucléotide en position 1 jusqu'à un nucléotide compris entre les positions 2056 et 2129 inclus de la séquence SEQ ID NO: 1.

13. Acide nucléique codant pour une protéine d'enveloppe GALV, caractérisé en ce qu'il comprend une séquence s'étendant du nucléotide en position 1 jusqu'à un nucléotide compris entre les positions 2056 et 2112 inclus de la séquence SEQ ID NO: 1.

14. Acide nucléique codant pour une protéine d'enveloppe GALV, caractérisé en ce qu'il comprend une séquence s'étendant du nucléotide en position 1 jusqu'au nucléotide 2058 de la séquence SEQ ID NO: 1.

15. Vecteur comprenant un acide nucléique selon l'une des revendications 11 à 14.

16. Cellule comprenant un acide nucléique selon l'une des revendications 11 à 14 ou un vecteur selon la revendication 15.

17. Cellule de packaging de rétrovirus, caractérisée en ce qu'elle comprend un acide nucléique selon l'une des revendications 11 à 14 ou un vecteur selon la revendication 15 et un acide nucléique codant pour les protéines gag et pol d'un rétrovirus.

18. Cellule selon les revendications 16 ou 17, caractérisée en ce qu'il s'agit d'une cellule de mammifère, de préférence humaine.

19. Utilisation d'un acide nucléique selon l'une des revendications 11 à 14 ou un vecteur selon la revendication 15 pour la production d'une cellule de packaging de rétrovirus.

20. Procédé de production de rétrovirus recombinants défectifs, comprenant l'introduction, dans une cellule selon la revendication 17, d'un vecteur rétroviral, et la récupération des virus recombinants produits.

21. Utilisation d'une protéine d'enveloppe fusogène pour la préparation d'une composition destinée à la mise en oeuvre d'une méthode de traitement thérapeutique et/ou vaccinal, notamment au traitement de cellules humaines.

22. Utilisation d'une protéine d'enveloppe fusogène pour la destruction de cellules humaines in vitro.

23. Utilisation d'une protéine d'enveloppe fusogène pour la préparation d'hybridomes in vitro.

24. Utilisation selon les revendications 21 à 23, caractérisé en ce qu'il s'agit d'une protéine d'enveloppe rétrovirale modifiée dans la région C-terminale, notamment par délétion de tout ou partie du domaine cytoplasmique ou d'une protéine d'enveloppe chimérique comprenant un tel domaine C-terminal modifié.

25. Procédé de préparation d'une protéine fusogène, comprenant (i) la modification de la région cytoplasmique d'une protéine d'enveloppe, notamment rétrovirale, et (ii) la mise en évidence de l'activité fusogène de la protéine.
